# EUROPEAN PATENT SPECIFICATION

(11) **EP 1 637 063 B1**
(45) Date of publication and mention of the grant of the patent: **28.11.2012**
(21) Application number: 04746697.4
(22) Date of filing: 23.06.2004
(51) Int. Cl.: A61B 1/00

(54) **CAPSULE TYPE MEDICAL DEVICE COMMUNICATION SYSTEM**
KOMMUNIKATIONSSYSTEM FÜR EIN MEDIZINPRODUKT VOM KAPSELTYP
SYSTEME DE COMMUNICATION DE DISPOSITIF MEDICAL DE TYPE GELULE

(30) Priority: 24.06.2003 JP 2003179647; 01.06.2004 JP 2004162986
(43) Date of publication of application: 22.03.2006
(60) Divisional application: 10010554.3
(73) Proprietor: Olympus Corporation, Tokyo 151-0072 (JP)
(72) Inventor: YOKOI, Takeshi, Hino-shi, Tokyo 191-0042 (JP); HOMAN, Masatoshi, Hino-shi, Tokyo 191-0062 (JP); FUJITA, Manabu, Hino-shi, Tokyo 191-0003 (JP); UCHIYAMA, Akio Oosone-town-house 3-303, Yokohama-shi, Kanagawa-ken, (JP); TAKIZAWA, Hironobu, Hachioji-shi, Tokyo 193-0844 (JP); KIMOTO, Seiichiro, Hachioji-shi, Tokyo 192-0045 (JP)
(74) Representative: von Hellfeld, Axel
(86) International application number: PCT/JP2004/009228
(87) International publication number: WO 2004/112592

(56) References cited:
- WO-A-03/060650
- JP-A- 2003 135 389
- JP-A- 2004 167 163
- US-A1- 2003 114 897
- US-B1- 6 493 587

## Description

### BACKGROUND OF THE INVENTION

Priority is claimed on Japanese Patent Application No. 2003-179647, filed June 24, 2003 and Japanese Patent Application No. 2004-162986, filed June 1, 2004, the contents of which are incorporated herein by reference.

### Field of the Invention

The present invention relates to a capsule type medical apparatus communication system, a capsule type medical apparatus, and an information receiver which are used for inspection or the like within a body cavity.

### Description of Related Art

In recent years, in the field of endoscopes which observe the interior of the alimentary canal of an examinee (a patient), there have been various proposals for capsule endoscopes of a type which is swallowed (for example, refer to Japanese Unexamined Patent Application, First Publication No. H01-305925 and Japanese Unexamined Patent Application, First Publication No. H04-109927). These capsule endoscopes are endowed with an image formation function and a wireless communication function.

When a capsule endoscope is swallowed by an examinee, along with proceeding within his body along with the peristalsis of his alimentary canal, during this time, it transmits images of the living body under examination at a fixed frame rate by a wireless signal. These transmitted images of the body of the patient under examination are received and recorded by a receiver which the examinee has attached to his body. And, after the capsule endoscope has been excreted, a doctor or a nurse downloads the image data which has been recorded in the receiver to a workstation, and performs diagnosis by looking at the images of the body of the patient under examination which are displayed upon the screen of the workstation.

Furthermore, with a prior art capsule endoscope, the wireless signals are transmitted in one direction only. In other words, the structure is such that the capsule endoscope is only endowed with a signal transmission function, and the receiver is only endowed with a signal reception function. Yet further, the structure is generally such that the capsule endoscope which has been introduced into the body of the patient under examination continues to transmit the image signals by wireless regardless of the state of reception of signals by the receiver.

On the other hand, as methods for a patient to confirm his own state of health, various types of inspection such as inspection by a medical examination or endoscope inspection or the like are generally known. Furthermore, as described above, an inspection method with a capsule type medical apparatus which easily performs inspection of the state of health by swallowing an inspection device which is formed as a capsule (a capsule endoscope) is known. Although various types of this kind of capsule type medical apparatus are proposed, as one concrete example, there is known an electronic endoscope device which, along with forming images of various portions of the interior of a human body, transmits these images of the interior of a human body which have been formed, to an image monitor section which is disposed outside the human body (for example, refer to Japanese Unexamined Patent Application, First Publication No. H04-109927).

This electronic endoscope device comprises an image formation head section which, within the body of the patient, forms images of various portions within the body of the patient, and a and an image monitor section which is disposed outside the body of the patient. With in a capsule shaped external surrounding container which is made from plastic or the like, the image formation head section includes: an objective lens which forms images of various portions within the body of the patient; a solid image formation element such as a CCD chip or the like; an image processing circuit element which processes the image signals from this solid image formation element; an integrated circuit for signal transmission which transmits the image signals which have been processed by this image processing circuit element to an image monitoring circuit outside the body of the patient as a radio wave; and a battery which supplies electrical power to a printed antenna for this signal transmission and to various other sections. Furthermore, these solid image formation element, image processing circuit element, integrated circuit for signal transmission, printed antenna, and battery are mutually connected together by a circuit board.

When performing inspection with this electronic endoscope device, first, in the vicinity of the image monitoring section, the patient swallows the image formation head section. This image formation head section which has been thus introduced within the body of the patient forms images of various portions within his body with the solid image formation element. The informed images are subjected to a predetermined processing by the image processing circuit element, and, thereafter, image signals are sent by the integrated circuit for signal transmission to the image monitor section via the printed antenna. And inspection is performed based upon these image signals which have thus been sent and have arrived.

Furthermore, as another capsule type medical apparatus, there is known a information recording capsule which measures information such as the temperature, the humidity, the pH, the pressure, or the like within the body of the patient over a long time period, and which, along with recording this information, sends it to outside the body of the patient (for example, refer to Japanese Unexamined Patent Application, First Publication No. H01-305925).

This information recording capsule includes: a sensor which measures the temperature, the pH, the pressure, or the like within the body of the patient and converts them into an electrical signal; a memory which stores the signal from this sensor; a data processing section such as a MPU (Micro Processing Unit) or the like which performs predetermined processing of the information data which has been sent from the sensor and has arrived; a timer including a clock signal generator which supplies a reference clock signal for the data processing section to perform time measurement; and a micro signal receiver which controls the data processing section based upon signals from outside the body of the patient and a micro signal transmitter which transmits the information as an ultrasonic wave to a signal reception device or the like outside the body of the patient.

When performing inspection by using this information recording capsule, after the patient has swallowed the information recording capsule, a signal is sent from a micro capsule control device outside the body of the patient to the micro signal receiver within the information recording capsule. When the micro signal receiver receives the signal, along with operating the data processing section, it stores in the memory the day of measurement, the time of measurement, and the like from the timer, in correspondence with the operational timing of this data processing section. Furthermore, the data processing section detects the information such as the pH and the like by operating the sensor, and performs the data processing. The data processing section stores the data after data processing in the memory section. In other words, the memory section stores the data which has been data processed in correspondence with the day and the time at which it was measured. And information is sent to the external signal receiver outside the body of the patient by the micro transmitter, and then is inspected.

Document US 2003/114897 A1 concerns a communication means for establishing a communication session between a "master" and a "slave", wherein, first, the master transmits a digital key to the slave and then the slave transmits a response to the master if the slave receives the digital key.

Document US 6,493,587 concerns a communication means for preventing an unauthorized access to an implanted component from an external component in which each of the implanted component and the external component is provided with a key and a lock which are used to perform authorization between the implanted component and the external component when they communicate with each other.

### SUMMARY OF THE INVENTION

Some or all of the above problems are overcome by a capsule type medical apparatus communication system having the features of claim 1.

A first capsule type medical apparatus communication system includes a capsule type medical apparatus communication system, including a capsule type medical apparatus which transmits information relating to the body of an examinee from within the body of the examinee to outside it, and an information receiver which is disposed externally to the body of the examinee and which receives the information, wherein: the capsule type medical apparatus transmits towards the information receiver a communication confirmation signal which confirms the state of communication with the information receiver; the information receiver, when it has received the communication confirmation signal, transmits a communication authorization signal which authorizes the capsule type medical apparatus to perform communication; and the capsule type medical apparatus includes a communication control device which transmits the information when it has received the communication authorization signal.

The communication authorization signal may also serve as a wireless signal which performs supply of electrical power to the capsule type medical apparatus.

The first capsule type medical apparatus includes: an acquisition device which acquires information about the body of an examinee; a signal transmission device which transmits, towards an information receiver which is disposed externally to the body of the examinee, the information which has been acquired, and a communication confirmation signal which confirms the state of communication with the information receiver, a signal reception device which, if the communication confirmation signal has been received, receives a wireless signal which has been transmitted from the information receiver which includes at least a communication authorization signal; and a communication control device which makes a decision as to whether or not to transmit the information, based upon the state of reception of the communication authorization signal.

The same antenna may be used for both the signal transmission device and the signal reception device.

The signal reception device may include an envelope wave detection circuit.

The signal reception device may include a rectification circuit which obtains electrical power from the wireless signal, and a communication authorization detection section which detects the communication authorization signal from the output of the rectification circuit and sends it to the communication control device.

The information receiver of the present invention includes: a signal reception device which receives, from a capsule type medical apparatus within the body of an examinee, information about the body of the examinee which has been transmitted, and a communication confirmation signal which confirms the state of communication with the capsule type medical apparatus; a recording section which records the information which has been received; a communication authorization signal creation section which, when the signal reception device has received the communication confirmation signal, creates a communication authorization signal which authorizes the capsule type medical apparatus to transmit the information; and a communication authorization signal transmission device which transmits the communication authorization signal.

The communication authorization signal may also serve as a wireless signal which performs supply of electrical power to the capsule type medical apparatus.

When the communication confirmation signal is not received, the communication authorization signal transmission device may transmit the communication authorization signal over an interval which is longer than the signal transmission interval of the communication confirmation signal.

A second capsule type medical apparatus includes, in a container which is introduced to within a living body: an acquisition device which acquires information about the interior of the living body; a signal transmission device which transmits the information which has been acquired by the acquisition device to outside the living body; a sensor which detects information which specifies the position and/or the attitude of the container within the living body; an inference device which infers the state of communication with outside the living body based upon the information which has been detected by the sensor; and a signal transmission control device which controls the signal transmission device, based upon the results of inference by this inference device.

As the sensor, a magnetic sensor which detects magnetic direction may be used.

As the sensor, a giro which detects the orientation of the container may be used.

As the sensor, a gravity sensor which detects the direction of gravity may be used.

As the sensor, a luminance sensor which detects the luminance within the living body may be used.

As the sensor, a pH sensor which detects the pH value within the living body may be used.

The second capsule type medical apparatus communication system includes the second capsule type medical apparatus, and an antenna external to the living body, which is disposed outside the living body, and which receives the information which is transmitted from the signal transmission device; and, here, the sensor is a signal reception antenna which receives the radio wave which is transmitted from the antenna external to the living body.

A third capsule type medical apparatus communication system includes: the second type of capsule type medical apparatus; an antenna external to the living body, which is disposed outside the living body, and which receives the information which is transmitted from the signal transmission device; and an energy wave transmission device which is disposes in the vicinity of the antenna external to the living body, and which transmits an energy wave towards within the living body; and, here, the sensor is an energy wave reception device which receives the energy wave which has been transmitted from the energy wave transmission device; and the signal transmission device performs signal transmission using electrical power which has been converted from the energy wave.

A fourth capsule type medical apparatus communication system includes: the second capsule type medical apparatus, of which the sensor is a magnetic sensor; an antenna external to the living body, which is disposed outside the living body, and which receives the information which is transmitted from the signal transmission device; and a magnet which is arranged in a direction which is correlated with a signal reception direction of the antenna external to the living body.

A fifth capsule type medical apparatus communication system includes: the second capsule type medical apparatus, of which the sensor is a giro; and an antenna external to the living body, which is disposed outside the living body, and which, along with receiving the information which is transmitted from the signal transmission device, also has a signal reception direction in a predetermined direction.

A sixth capsule type medical apparatus communication system includes: the second capsule type medical apparatus, of which the sensor is a luminance sensor; and an antenna external to the living body, which is disposed outside the living body, and which receives the information which is transmitted from the signal transmission device.

With the above described second through sixth capsule type medical apparatus communication systems, the antenna external to the living body may be arranged in a state in which it is separated from the surface of the living body by a predetermined interval distance.

In this case, the antenna external to the living body may move in a state in which the predetermined interval distance is maintained.

### BRIEF DESCRIPTION OF THE DRAWINGS

FIG. 1 is an overall structural view showing a capsule type medical apparatus communication system according to the first embodiment of the present invention.
FIG. 2 is a sectional view of a capsule endoscope which is a structural element of the capsule type medical apparatus communication system.
FIG. 3 is a block diagram showing the structure of a receiver which is another structural element of the capsule type medical apparatus communication system.
FIGS. 4A and 4B are flow charts showing the communication operation between a capsule endoscope and a receiver, when obtaining information relating to an examinee by the capsule type medical apparatus communication system.
FIG. 5 is a sectional view of a capsule endoscope according to a second embodiment of the present invention.
FIG. 6 is a circuit diagram of and around a signal reception antenna in the capsule endoscope.
FIG. 7 is a sectional view of a capsule endoscope according to a third embodiment of the present invention.
FIG. 8 is a block diagram of the capsule endoscope.
FIG. 9 is a general view showing a capsule endoscope communication system and a capsule endoscope according to a first example.
FIG. 10 is a figure showing the state of communication by the capsule endoscope communication system with a capsule endoscope within a human body.
FIG. 11 is a sectional view of the capsule endoscope.
FIG. 12 is a view showing an air mat which includes an antenna external to the body which is used in the capsule endoscope communication system.
FIG. 13 is a sectional view of abdominal of an examinee; when the air mat is fitted to the vicinity of the abdomen of the patient.
FIG. 14 is a sectional view of abdominal of an examinee, showing the situation in which the capsule endoscope is positioned in the stomach of the examinee.
FIG. 15 is a flow chart when communicating with the capsule endoscope with this capsule endoscope communication system.
FIG. 16 is a general view showing a capsule endoscope communication system and a capsule endoscope according to a second example.
FIG. 17 is a view showing the situation of communication by the capsule endoscope communication system with the capsule endoscope within the body of an examinee.
FIG. 18 is a sectional view of the capsule endoscope.
FIG. 19 is a general view showing a capsule endoscope communication system and a capsule endoscope according to a third example.
FIG. 20 is a view showing the situation of communication by the capsule endoscope communication system with the capsule endoscope within the body of an examinee.
FIG. 21 is a sectional view of the capsule endoscope.
FIG. 22 is a general view showing a capsule endoscope communication system and a capsule endoscope according to a fourth example
FIG. 23 is a sectional view of the capsule endoscope.
FIG. 24 is a general view showing a capsule endoscope communication system and a capsule endoscope according to a fifth example.
FIG. 25 is a sectional view of the capsule endoscope.
FIG. 26 is a general view showing a capsule endoscope communication system and a capsule endoscope according to a sixth example.
FIG. 27 is a figure showing a mat which is used in the capsule endoscope communication system.
FIG. 28 is a sectional view of abdominal of an examinee, showing a patient when this mat is fitted to that patient.
FIG. 29 is a general view showing a capsule endoscope communication system and a capsule endoscope according to a seventh example
FIG. 30 is a frontal view showing a shifting mechanism which is a structural element of a communication device which is used in this capsule endoscope communication system, and which is provided within a chair.
FIGS. 31A and 31B show a variant example of the communication device shown in FIG. 29; FIG. 31A is a side view of the chair, and FIG. 31B is a frontal view of an antenna array.
FIG. 32 shows a variant example of the air mat shown in FIG. 12, and is a figure showing the state in which this mat is fitted to an examinee.
FIG. 33 is a perspective view showing the mat.
FIG. 34 is a view showing a variant example of the mat.

### DETAILED DESCRIPTION OF THE INVENTION

### (First embodiment)

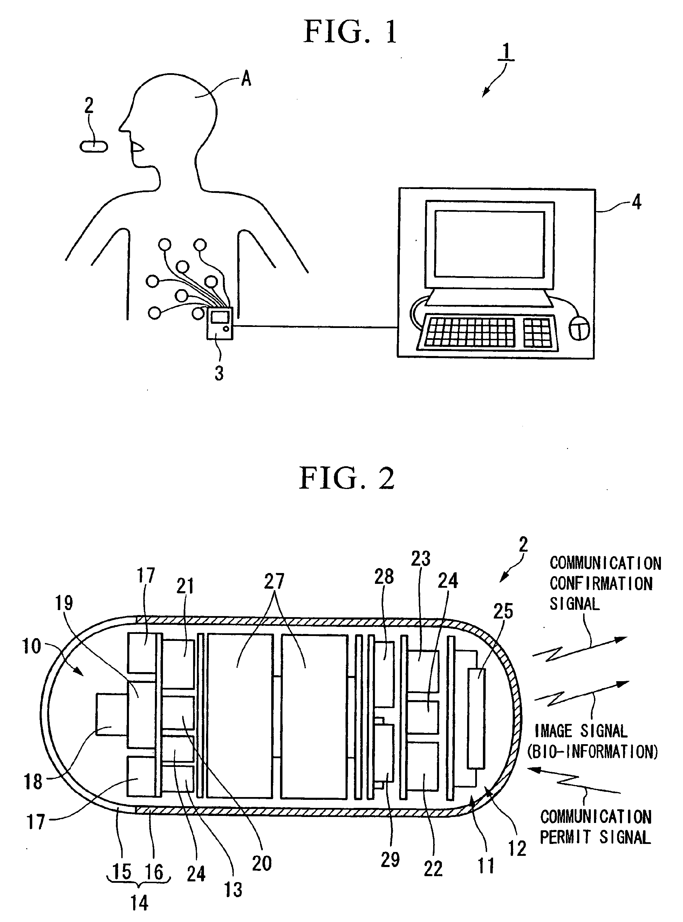

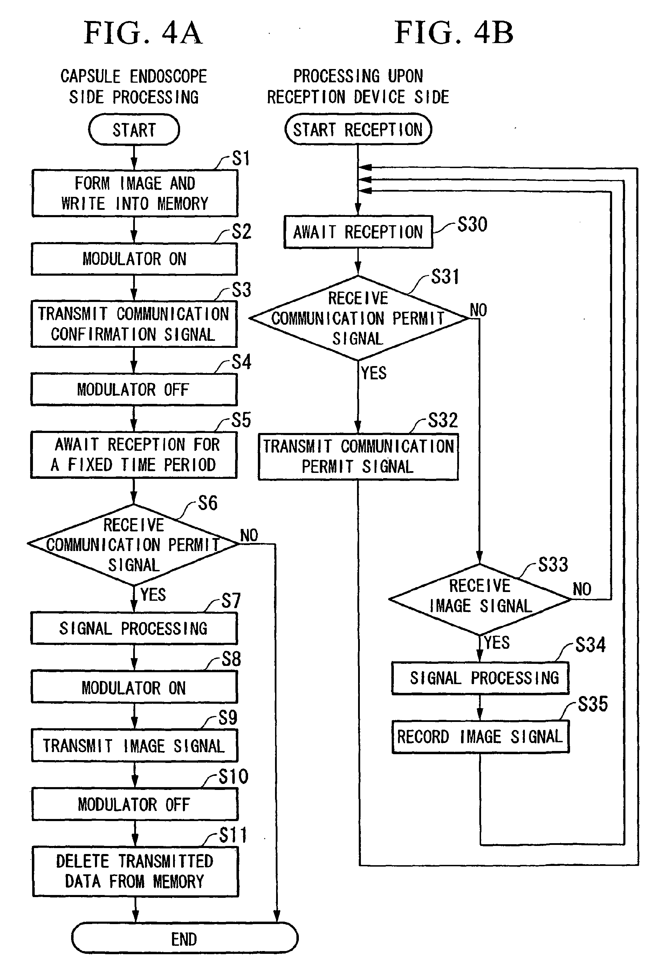

In the following, a capsule type medical apparatus communication system, a capsule type medical apparatus, and an information receiver according to the first embodiment of the present invention will be explained with reference to FIGS. 1 through 4A and 4B.

As shown in FIG. 1, the capsule type medical apparatus communication system 1 according to the present embodiment is capable of detecting information, and includes: a capsule endoscope (a capsule type medical apparatus) 2 which transmits information about a examinee A (i.e., about the body of an examinee) from within the body of the examinee to outside that body; a receiver (an information receiver) 3 which is provided externally to the body of the examinee, and which receives an image signal, which constitutes the information, which has been transmitted from the capsule endoscope 2; and a workstation 4 for displaying image data which has been recorded in this receiver 3.

The capsule endoscope 2 transmits a communication confirmation signal for confirming its state of communication with the receiver 3, and the receiver 3, when it has received this communication confirmation signal, transmits a communication authorization signal for authorizing the communication of the information. Furthermore, the capsule endoscope 2 transmits the information, when it has received the communication authorization signal. The details of these matters will be explained hereinafter.

The above described capsule endoscope 2 is one which can be swallowed by an examinee A, and it is thus introduced to within the body of this examinee A and obtains (detects) the information. Moreover, in the present embodiment, the case will be explained in which the above described information is an image (an image signal) of the interior of the alimentary canal of the examinee A.

The capsule endoscope 2 of the present embodiment, as shown in FIG. 2, includes an acquisition device 10 which acquires an image (the information), a signal transmission device 11 which transmits an image which has been acquired and the above described communication confirmation signal to the receiver 3, a signal reception device 12 which receives a wireless signal including at least the above described communication authorization signal which has been transmitted from the receiver 3 upon receipt of the communication confirmation signal, and a communication control section (a communication control device) 13 which decides whether or not to transmit images, based upon the state of reception of the communication authorization signal. These structural elements are housed within a casing 14.

The casing 14 includes a capsule shaped container 15 and a transparent cover 16 which is made from a transparent material for image formation. Furthermore, in the interior of the casing 14, there are housed: LEDs 17 for illuminating the interior of the body of the patient; an objective lens 18 which forms an image of the interior of the body of the patient, in other words, which forms an image of the region within the body of the patient which is the subject of observation; a solid-state imager 19 which forms an image of the interior of the body of the patient; a memory 20 which stores the image which has been formed; an image formation section control device 21 which controls the above described LEDs 17 and the above described solid-state imager 19; a modulator 22 which modulates the image signal for transmission; a demodulator 23 which demodulates the above described communication authorization signal, which is a control signal which is transmitted from the receiver 3 by wireless; a signal processing circuit 24 which changes the image signal which has been formed by the solid-state imager 19 into an appropriate format for transmission, and performs predetermined processing upon the above described communication authorization signal; an antenna 25 which performs transmission and reception of various types of signal between the above described communication control section 13 and the receiver 3; a changeover switch 26 which performs connection changeover so as to connect one or another of the modulator 22 and the demodulator 23 to the antenna 25; a battery 27 and a power source circuit 28 which supply power to each of the abovementioned structural elements; and a power source switch 29 which controls whether or not the capsule endoscope 2 is operated.

The LEDs 17, the objective lens 18, the solid-state imager 19, and the image formation section control device 21 constitute the above described acquisition device 10. Furthermore, the antenna 25, the changeover switch 26, and the modulator 22 constitute the above described signal transmission device 11, while the antenna 25, the changeover switch 26, and the demodulator 23 constitute the above described signal reception device 12. Yet further, as described above, the signal transmission device 11 and the signal reception device 12 both use the same antenna 25 and change it over by the changeover switch 26.

The above described objective lens 18 is provided upon the interior side of the transparent cover 16, and the above described solid-state imager 19 such as a CCD imager or the like, is disposed at the image focusing position of the objective lens 18. Furthermore, the above described LEDs 17 which are, for example white in color are arranged in a plurality around the periphery of the objective lens 18 as illumination elements. Moreover, the solid-state imager 19 converts the image which has been focused by the objective lens 18 into an electronic signal, and the image which has been formed by this solid-state imager 19 is subjected to predetermined processing such as image processing or the like by the signal processing circuit 24, and then is transmitted from the antenna 25 after having been sent to the above described modulator 22 (which is disposed at the rear portion of the capsule endoscope 2).

The antenna 25 also has a role of a signal reception antenna which receives the communication authorization signal, which is the control signal from the receiver 3. When the communication state between the capsule endoscope 2 and the receiver 3 is good, the communication authorization signal which has been transmitted from the receiver 3 to the capsule endoscope 2 is received by the antenna 25. This communication authorization signal which has been received is transmitted to the communication control section 13 after having been demodulated by the demodulator 23. And, along with recognizing this communication authorization signal which has been transmitted, the communication control section 13 also, based upon the results thereof, makes a decision as to whether or not to transmit an image as a signal, and performs control of the above described signal transmission device 11.

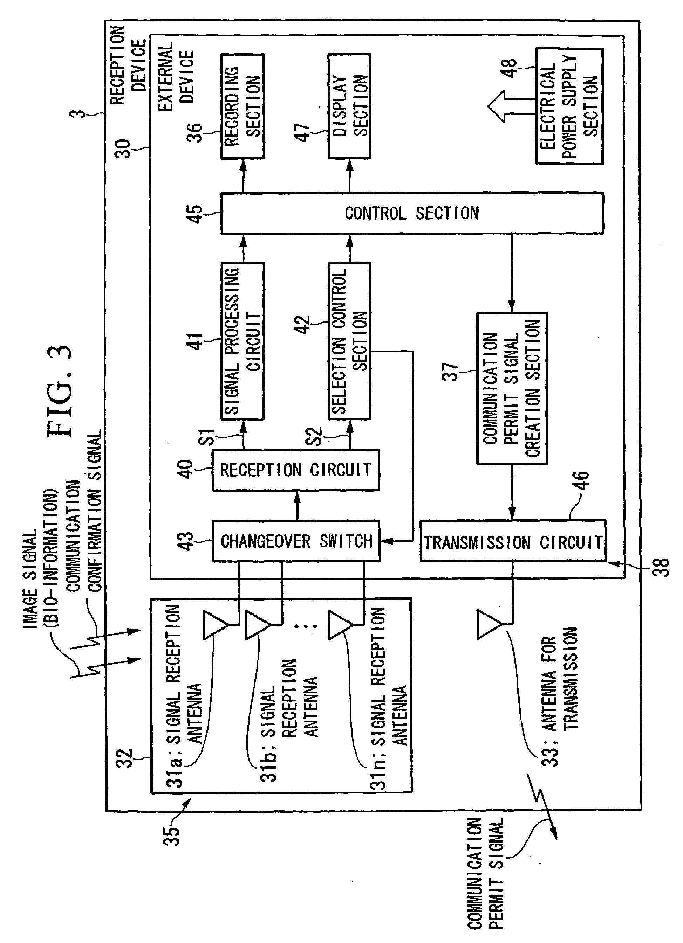

The above described receiver 3, as shown in FIG. 3, includes: an external device 30 which performs demodulation and recording and the like of the various signals which have been transmitted from the capsule endoscope 2 (the image signal or the communication confirmation signal); a signal reception antenna unit 32 which includes a plurality of signal reception antennas 31a, 31b, .... for receiving the various signals; and a signal antenna for transmission 33 for transmitting the communication authorization signal.

Furthermore, the receiver 3 of the present embodiment includes: a signal reception device 35 which receives the above described image signal (the information) and communication confirmation signal; a recording section 36 which records the image signal which has been received; a communication authorization signal creation section 37 which creates a communication authorization signal which, when the signal reception device 35 has received the communication confirmation signal, authorizes the capsule endoscope 2 to transmit a signal containing the information; and a communication authorization signal transmission device 38 which transmits the communication authorization signal which has been created.

Each of the signals which have been received by the above described signal reception antenna unit 32 is demodulated by a signal reception circuit 40. The demodulated output S 1 which is outputted from this signal reception circuit 40 is transmitted to a signal processing circuit 41, and processing is performed according to the type of the signal. Furthermore, a received signal intensity signal S2 which is outputted from the signal reception circuit 40 is transmitted to a selection control section 42. From the received signal intensity signal S2 which has been transmitted, the selection control section 42 compares together the signal reception intensity for the various antennas 31 a, 31 b, ... for signal reception, and then selects an antenna which is the most suitable for signal reception. And, based upon the result of selection, the selection control section 42 controls the changeover switch 43 to perform actual antenna changeover.

If the signal which has been received by the signal reception antenna unit 32 is an image signal which has been transmitted from the capsule endoscope 2, then it is subjected to processing by the signal processing circuit 41 such as adjustment of the image data, compression, and the like, and the image data after the processing is recorded in the recording section 36 via the control section 45. As the recording section 36, for example, a transportable recording medium may be used.

On the other hand, if the signal which has been received is a communication confirmation signal which has been transmitted from the capsule endoscope 2, then, after the control section 45 has recognized the communication confirmation signal, this control section directs the communication authorization signal creation section 37 to generate a communication authorization signal which authorizes the capsule endoscope 2 to transmit an image signal. The communication authorization signal which has been created by the communication authorization signal creation section 37 is transmitted from the antenna 33 for signal transmission after having been modulated by the signal transmission circuit 46.

Furthermore, the various types of information such as information about the examinee A (the patient), error information, and the like are displayed upon a display section 47 and the workstation 4 by the control of the control section 45. Yet further, the electrical voltages which are required by each of the functional blocks of the receiver 3 are supplied from an electrical power supply section 48.

The above described signal-reception circuit 40, signal processing circuit 41, selection control section 42, changeover switch 43, control section 45, recording section 36, communication authorization signal creation section 37, signal transmission circuit 46, display section 47, and electrical power supply section 48 constitute the above described external device 30.

Furthermore, the signal reception antenna unit 32, the changeover switch 43, and the signal reception circuit 40 constitute the above described signal reception device 35, while the antenna 33 for signal transmission and the signal transmission circuit 46 constitute the above described communication authorization signal transmission device 38.

The acquisition of the information for an examinee A, in other words, the acquisition of an image of the interior of his alimentary canal, by the capsule type medical apparatus communication system 1 constituted in this manner will now be explained with reference to FIGS. 4A and 4B.

The capsule endoscope 2 which has been introduced to within the body of the examinee A performs image formation operation at a timing which is determined by the image formation section control device 21, and the image data which has been formed is recorded into the memory 20 (S 1). After this, the capsule endoscope 2 turns the modulator 22 ON (S2), and transmits a communication confirmation signal for deciding whether or not the state of communication with the receiver 3 is satisfactory (S3).

Moreover, since the intensity of the communication confirmation signal is roughly equal to the intensity when transmitting the image signal, accordingly, when it is possible to receive the communication confirmation signal, the receiver 3 makes a decision that it is also possible to receive the image signal. Furthermore, it is desirable for the communication confirmation signal to be made up according to a fixed pattern, so that the receiver 3 can distinguish it from external noise which it receives. However, the format of the communication control signal is not limited to this; for example, it would also be acceptable to utilize a non modulated signal as the communication confirmation signal, and to decide whether or not the communication confirmation signal has been transmitted by checking the intensity of signal reception on the side of the receiver 3.

When the transmission of the above described communication confirmation signal has been completed, then the modulator 22 is turned into the OFF state (S4).

On the other hand, the receiver 3 goes into a signal reception waiting state until it receives a communication confirmation signal or an image signal (S30). If a communication confirmation signal is received (YES in S31) during the signal reception wait, then the receiver 3 transmits a communication authorization signal to the capsule endoscope 2 (S32). Moreover, it is desirable for the communication authorization signal, just like the communication confirmation signal, to be made up from a fixed pattern, so that it can be distinguished from external noise. However, the format of the communication authorization signal is not limited to this.

And, after having transmitted the communication authorization signal, the receiver 3 again returns to the signal reception wait state (S30).

After transmitting the communication confirmation signal (S3), the capsule endoscope 2 goes into the signal reception wait state (S5) for a fixed interval until it is anticipated that a communication authorization signal will come back. If, in this interval, a communication authorization signal has been received (YES in S6), then a decision is made as to whether or not the communication control section 13 is to transmit an image signal, and if it is decided that such signal transmission is taking place then control of the signal transmission device 11 is performed so as to transmit the image signal; in other words, along with converting the image data which has been stored in the memory 20 to image data for signal transmission which is suitable for signal transmission (S7) by the signal processing circuit 24, the modulator 22 is put into the ON state by control by the signal processing circuit 24 (S8). And, after the image data for signal transmission has been modulated by the modulator 22, it is transmitted from the antenna 25 (S9). Furthermore, after the image data for signal transmission has been transmitted, the modulator 22 again goes into the OFF state (S 10), and also the image data is deleted from the memory 20 (S11).

On the other hand, if the communication authorization signal is not received within the fixed interval (NO in S6), then the modulator 22 stays in the OFF state just as it is, and awaits the next image formation timing.

When an image signal is transmitted from the capsule endoscope 2 (S9), the receiver 3, along with receiving the image signal by the signal reception device 12 (YES in S33), also performs prescribed processing such as image compression or the like with the signal processing circuit 24 (S34), and, along with recording the data after processing in the recording section 36 (S35), also displays it upon the display section 47 or the workstation 4.

Moreover, the image which is stored in the memory 20 of the capsule endoscope 2 is not limited to a single frame; it is also possible that a plurality of image frames are stored in the memory 20, and, when the communication authorization signal has been confirmed, this plurality of frames of image data are transmitted continuously.

By looking at these images which have been displayed, or at images which have been recorded in the recording section 36, a doctor or the like can diagnose the state of the health or the like of the examinee A.

As has been described above, according to the capsule type medical apparatus communication system 1, the capsule endoscope 2, and the receiver 3 of the present embodiment, it is possible to perform signal transmission or signal reception of image signals, which are the information, when the communication state between the capsule endoscope 2 and the receiver 3 is satisfactory. That is, before transmitting the image signals, the capsule endoscope 2 performs transmission of the communication confirmation signal. And, when the receiver 3 has received the communication confirmation signal, i.e. when the communication state of both sides is satisfactory, the receiver 3 transmits the communication authorization signal which authorizes communication of the image signals. Since the capsule endoscope 2 starts to transmit the image signals upon receipt of the communication authorization signal, accordingly, it is possible for the image signals to be reliably transmitted to the receiver 3. Accordingly, waste of electrical power does not take place, since the capsule endoscope 2 does not transmit image data (the image signals) when the receiver 3 cannot receive the image signals.

Furthermore, since the receiver 3 can obtain the image signals reliably, accordingly, there is no loss of acquired images due to poor communications. Due to this, it is possible to perform diagnosis of the state of health or the like of the examinee A in a reliable manner.

Furthermore if, for example, a structure is employed in which a plurality of frames of image data are stored in the memory 20, and they are all transmitted together when it is has been possible to receive the communication authorization signal, then it is possible to reduce the number of image frames or the like which are not received by the receiver 3, even though they have been transmitted from the capsule endoscope 2.

### (Second embodiment)

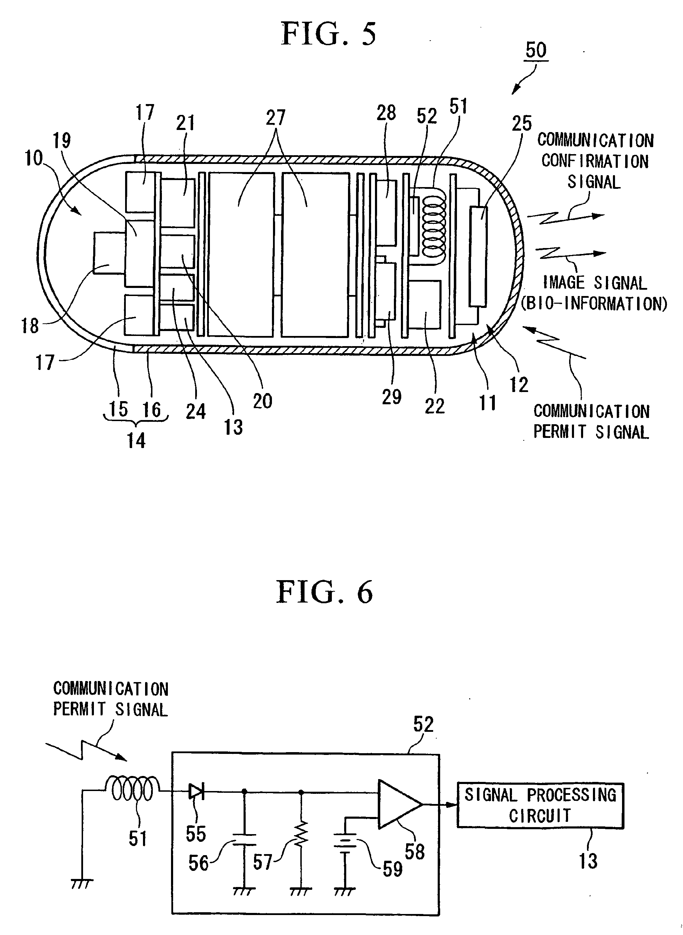

Next, a capsule type medical apparatus according to the second embodiment of the present invention will be explained with reference to FIGS. 5 and 6. Moreover, in the present embodiment, to structural elements which are the same as in the above described first embodiment, the same reference symbols will be affixed, and their explanation will be omitted.

The point of difference between the above described first embodiment and the present embodiment is that, by contrast with the capsule endoscope 2 of the above described first embodiment in which the common antenna 25 was employed when receiving the communication authorization signal, with the capsule endoscope (capsule type medical apparatus) 50 of the present embodiment, the signal reception device 12 includes a separate coil shaped antenna 51 which receives the communication authorization signal.

In other words, as shown in FIGS. 5 and 6, the capsule endoscope 50 of the present embodiment includes, within the casing 14, the above described coil shaped antenna 51, and a received signal detection circuit (an envelope wave detection circuit) 52 for detecting the communication authorization signal from the signal which has been received by this coil shaped antenna 51.

The operation of the above described received signal detection circuit 52 when the communication authorization signal has been transmitted from the receiver 3 to the capsule endoscope 50 constituted in the above described manner will now be explained referring to FIG. 6.

When the communication authorization signal reaches, an electrical potential with respect to the ground of the coil shaped antenna 51 as a reference is generated. And, when a sufficient electrical potential difference is generated between both ends of a diode 55, this diode 55 goes into the ON state, and electrical charge starts to accumulate in a condenser 56. Furthermore, a resistor 57 releases the electric charge which has accumulated in the condenser 56 to ground. Accordingly, the electrical potential at the upper end of the condenser 56 rises with a time constant which is determined by the values of the condenser 56 and the resistor 57. Furthermore, a comparator 58 compares together the electrical potential at the upper end of the condenser 56 and a standard electrical potential which is created by a reference voltage generator 59. In other words, it is possible to perform detection of the communication authorization signal by checking the output of the comparator 58.

As has been described above, the capsule endoscope 50 of the present embodiment is able to keep the consumption of electrical power low, since the greater portion of the received signal detection circuit 52 is made up from passive components. Furthermore, it is possible to make the device more compact, since the number of components is low.

Moreover, in the present embodiment, it is possible for the communication authorization signal which is transmitted from the receiver 3 to be kept small in comparison to the communication confirmation signal which is transmitted from the capsule endoscope 50, for example to around a few tens of KHz. By using this type of low frequency, it is possible to keep small the attenuation when it arrives from outside the body of the patient to within it.

### (Third embodiment)

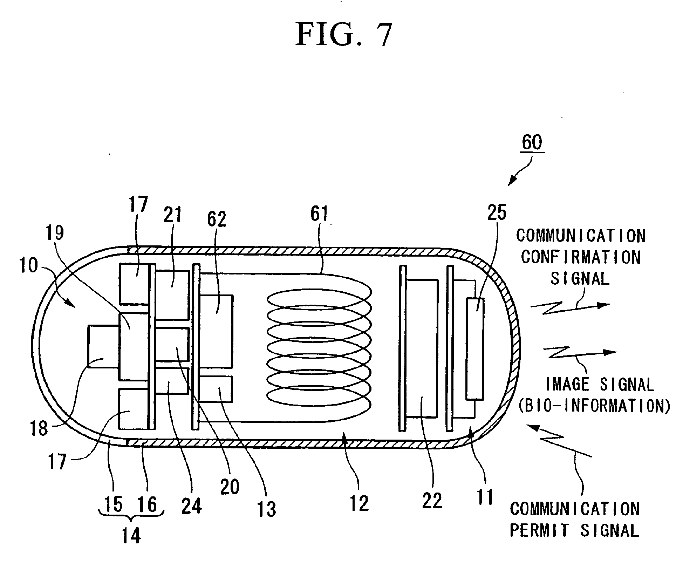

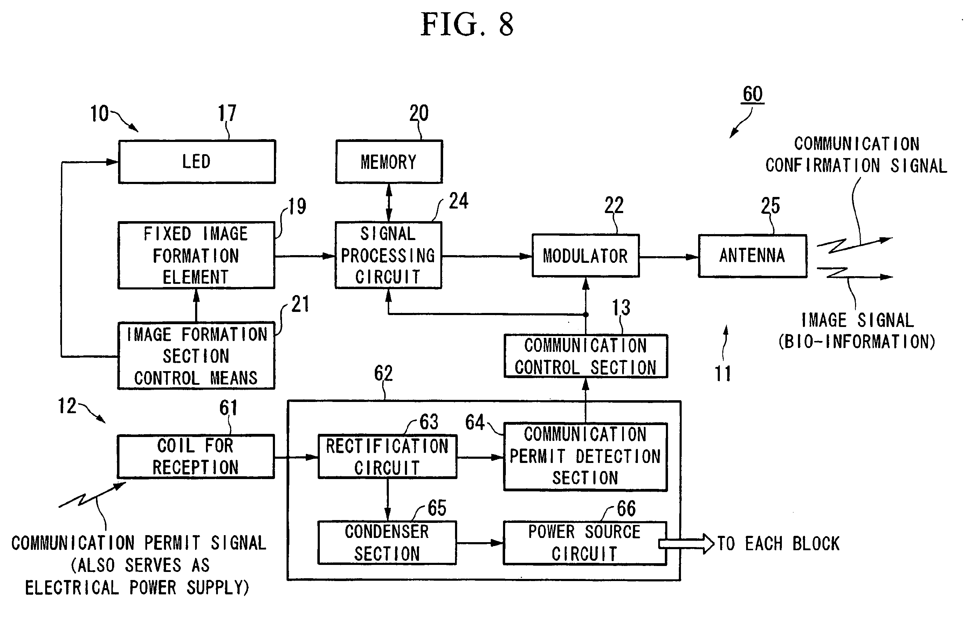

Next, a capsule type medical apparatus according to the third embodiment of the present invention will be explained with reference to FIGS. 7 and 8. It should be understood that, in the explanation of the present embodiment, to structural elements which are the same as in the above described first embodiment and the above described second embodiment, the same reference symbols will be affixed, and their explanation will be omitted.

The point of difference between the above described first embodiment and the present embodiment is that, by contrast with the capsule endoscope 2 of the above described first embodiment which was operated with a battery 27 which was internally housed, with the capsule endoscope (capsule type medical apparatus) 60 of the present embodiment, it is operated by receiving a supply of electrical power from the receiver 3 by wireless.

In other words, as shown in FIGS. 7 and 8, with the capsule endoscope 60 of the present embodiment, the signal reception device 12 includes a coil shaped antenna 61 for signal reception which receives electrical power which is transmitted from outside the body of the patient, and an electrical power signal reception section 62.

In the present embodiment, the communication authorization signal also serves as an electrical power signal (a wireless signal) for supplying electrical power to the capsule medical apparatus 60. It should be understood that the method of supply of electrical power is not to be construed as being limited to this one; for example, it would be acceptable to provide an electrical power supply device outside the body of the patient, and to supply electrical power to the capsule endoscope 60 by wireless from this electrical power supply device; or, along with the receiver 3 also serving as an electrical power supply device, to make it possible for the signal for electrical power supply to be transmitted from the antenna 33 for signal transmission separately from the communication authorization signal.

As shown in FIG. 8, the above described electrical power signal reception section 62 includes a rectification circuit 63 for obtaining electrical power from the communication authorization signal, and a communication authorization detection section 64 which detects the communication authorization signal from the output of this rectification circuit 63 and sends it to the communication control section 13. Moreover, the rectification circuit 63 has the same structure as the received signal detection circuit 52 of the second embodiment described above.

The operation of the electrical power signal reception section 62 when the communication authorization signal has been transmitted from the receiver 3 to the capsule endoscope 60 which possesses this type of structure will now be explained with reference to FIG. 8.

The communication authorization signal (which also serves for electrical power supply) which has been send from the receiver 3 is converted into a voltage by the coil shaped antenna 61 and is rectified by a rectification circuit 63. A communication authorization detection section 64 makes a decision as to whether or not the communication authorization signal is being transmitted from the output of the rectification circuit 63, and then sends the result thereof to the communication control section 13. Only if the communication authorization signal has been detected, the communication control section 13 operates the signal processing circuit 24 and the modulator 22 and transmits an image signal. Furthermore, the output of the rectification circuit 63 is temporarily accumulated in an accumulation section 65, and is supplied to the various structural elements after having been stabilized by a power source circuit 66.

Since, as has been described above, according to the capsule endoscope of the present embodiment, as it is possible to receive supply of electrical power via the communication authorization signal, it is possible to eliminate electrical power failures, such as elimination of the battery and the like. Thus, there is no problem with service life, and it is possible reliably to obtain an image of the interior of the body of the patient, which is the information. Furthermore, it is not necessary to provide any separate detection circuit, since the communication authorization detection section 64 detects the communication authorization signal from the output of the rectification circuit 63. Accordingly, the manufacture is easy, and it is possible to anticipate an enhancement of compactness. In particular, as the communication authorization signal also serves as the wireless signal for supplying an electrical power, only one transmission device is enough, and furthermore, the receiver 3 can be configured easily, and then the compactness can be improved.

In the present embodiment, since the capsule endoscope 60 is operated by receiving supply of electrical power from an external source, if the communication confirmation signal is not received by the receiver 3 from the capsule endoscope 60, two possibilities can be considered as a cause thereof. In other words, there are the following two possibilities: the possibility that the state of communication is not satisfactory, and the possibility that the capsule endoscope 60 is not operating due to insufficiency of electrical power.

In order to prevent insufficiency of electrical power for the capsule endoscope 60, when the communication confirmation signal is not received, it is desirable to set the communication authorization signal transmission device 38 of the receiver 3 so that the communication authorization signal is transmitted over an interval which is longer than the interval over which the communication confirmation signal is transmitted from the capsule endoscope 60. In this case, it is possible to supply electrical power to the capsule endoscope 60 in a timely manner, and it is possible to prevent non-transmission of the communication confirmation signal due to interruption of electrical power. Accordingly, it becomes possible reliably to obtain an image of the interior of the body of the patient.

### (First example)

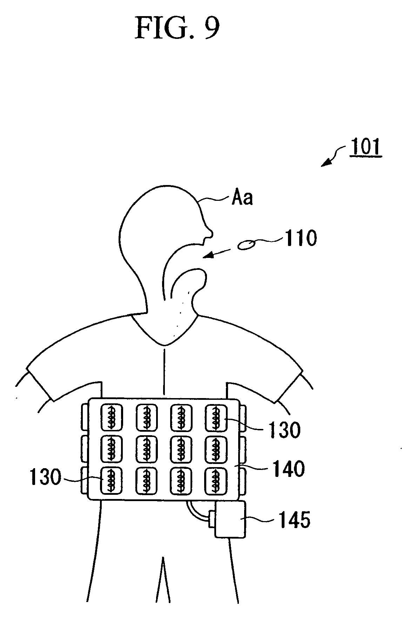

The capsule type medical apparatus and the capsule type medical apparatus communication system according to the first example will now be explained below with reference to FIGS. 9 through 15.

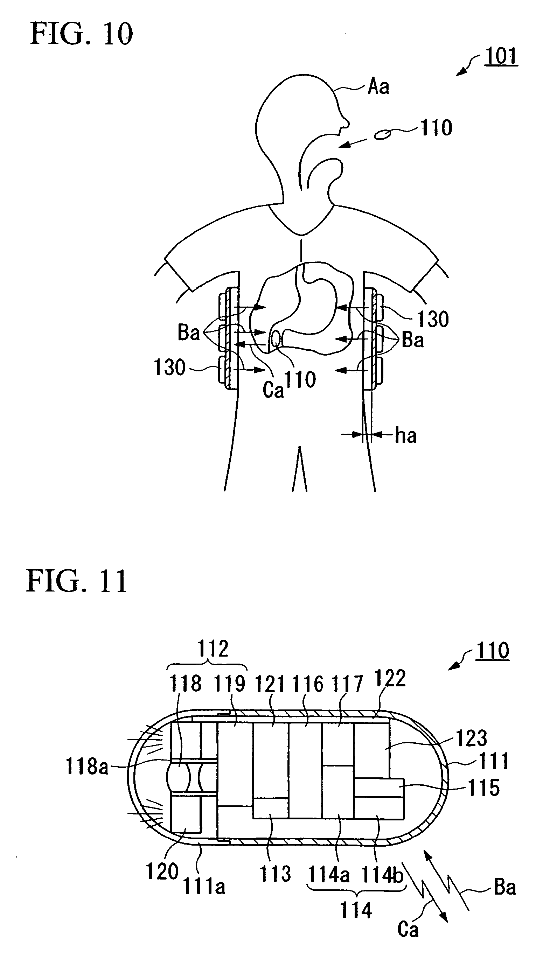

As shown in FIGS. 9 and 10, the capsule endoscope communication system (capsule type medical apparatus communication system) 101 includes a capsule endoscope (capsule type medical apparatus) 110 which can be swallowed by a patient Aa, and an antenna external to the body of the patient 130 which is disposed outside the body (outside the living body) and which transmits a radio wave Ba toward within the body of the patient.

The above described capsule endoscope 110 is introduced to within the body of the patient Aa and detects information (the information), and includes a capsule shaped vessel (container) 111 shown in FIG. 11. Within this vessel 111, there are housed: an image formation section (acquisition device) 112 which, by forming an image of the interior of the body of the patient, acquires the image which has been formed, which is the information; a memory 113 which stores the formed image which has been acquired by the image formation section 112; a signal transmission section (signal transmission device) 114 which transmits the formed image which has been stored in the memory 113 to outside the body of the patient; a signal reception antenna (sensor) 115 which detects a radio wave (information) Ba for specifying its own position and attitude; a comparison circuit (inference device) 116 which infers the communication state with an antenna external to the body of the patient 130 which is disposed externally to the body of the patient, based upon the radio wave Ba which have been detected by this signal reception antenna 115; and a signal transmission control section (signal transmission control device) 117 which controls the signal transmission section 114, based upon the results of inference by this comparison circuit 116.

The above described vessel 111 is made from a material such as plastic or the like, and is formed so that its interior is closed, while it is provided with a transparent cover 111 a at its one end side. With the transparent cover 111a, there is disposed an objective lens 118 which focuses an image of a subject body for observation, such as some region within the body of the patient or the like, and which is fitted into a lens frame 118a; and, at the position of the focused image, there is disposed an image formation element 119 which performs image formation, such as for example a CMOS imager or the like. This objective lens 118 and image formation element 119 constitute the above described image formation section 112.

Furthermore, as illumination elements, white colored LEDs 120, for example, are disposed around the objective lens 118. Moreover, on the rear side of the image formation element 119, there are disposed a processing section 121 which, along with driving the white colored LEDs 120, also performs driving of the image formation element 119 and processing of the image formation data and the like, and the above described memory 113. Furthermore, adjacent to the memory 113, there are also disposed the above described comparison circuit 116, signal transmission control section 117, signal transmitter 114a, signal transmission antenna 114b, and signal reception antenna 115. These structural elements described above are mutually connected together by a flexible printed circuit board 122, and the electrical power which they required is supplied by a battery 123. Furthermore, the signal transmitter 114a and the signal transmission antenna 114b constitute the above described signal transmission section 114.

The above described signal reception antenna 115 is endowed with the function of receiving the radio wave Ba which has been transmitted from the antenna external to the body of the patient 130, and of sending it to the comparison circuit 116. The comparison circuit 116, along with converting the radio wave Ba which has been transmitted into a signal value proportional to the level of the received signal, also compares this signal value and a threshold value which has been set in advance; and, if the value of the signal is the same value as or greater than the threshold value, infers that the state of communication with the antenna external to the body of the patient 130 is satisfactory. And the comparison circuit 116 has the function of transmitting the inference results which have been inferred to the signal transmission control section 117.

The signal transmission control section 117 performs the final determination as to whether or not to communicate, based upon the results of inference of the comparison circuit 116, then controls the signal transmitter 114a. For example, if the results of inference which have been sent from the comparison circuit 116 are the results showing the satisfactory communication states, then it operates the signal transmitter 114a. The signal transmitter 114a has the function of transmitting the formed image which is stored within the memory 113 as a signal to outside the body of the patient via the signal transmission antenna 114b as a radio wave Ca.

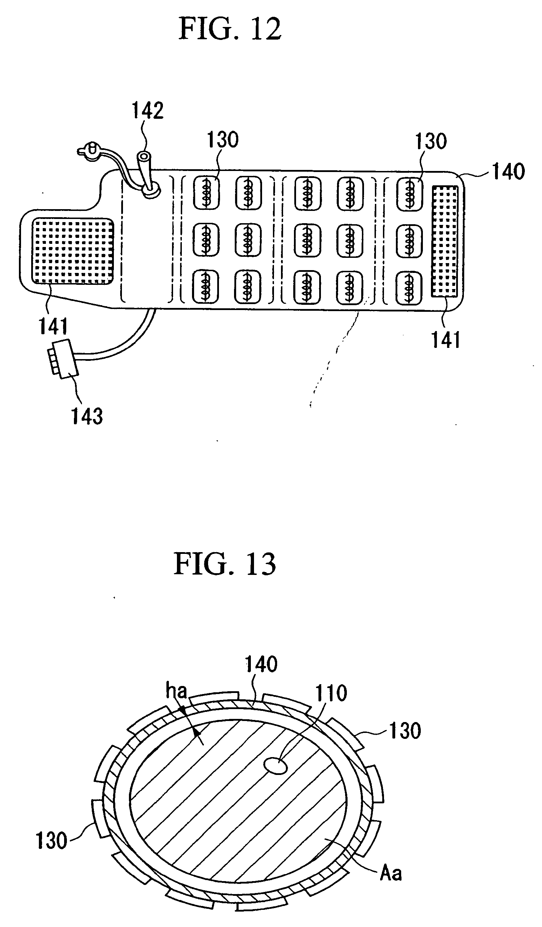

As shown in FIGS. 12 and 13, the above described antenna external to the body of the patient 130, for example, is fitted in plurality to an air mat 140 which can be attached to and removed from the patient Aa, so as to be spaced at a prescribed interval distance ha from the surface of the body of the patient.

The air mat 140 is made in the form of a belt, and connector 141 such as Velcro fasteners or the like are provided upon the front and rear surfaces of both end sides thereof. By this, it is possible for the patient Aa, for example, to wind it around the vicinity of his abdomen and fix it there. Furthermore, air can be blown into the interior of the air mat 140 from an air injection hole 142. In other words, the air mat 140 is fitted to the patient Aa by, after fixing it by winding it around the vicinity of his abdomen as has been described above, injecting air into its interior from the air injection hole 142. At this time, the outer side surface of the air mat 140 is in a state of being separated from the surface of the body of the patient Aa by just a prescribed interval distance ha.

Furthermore, a plurality of the above described antennas external to the body of the patient 130 are fitted upon the outer surface of the air mat 140 in agreement with the direction of the plane of polarization. Cables not shown in the figures are connected to these antennas external to the body of the patient 130, and these are all collected together into a plug portion 143, so that they can be connected to a recording device 145 which will be described later. Furthermore, the antennas external to the body of the patient 130 also have the function of receiving the radio wave Ca, i.e. of the formed image, which has been transmitted from the signal transmission section 114 of the capsule endoscope 110.

Yet further, the air mat 140 is made so that the recording device 145 shown in FIG. 9 may be fitted thereto. A receptacle not shown in the figures, to which the above described plug 143 can be connected, is provided to the recording device 145. By this, the recording device 145 is capable of recording a formed image which has been received by the antennas external to the body of the patient 130 in an internal memory not shown in the figures. It is possible to perform observation of the state of health of the patient Aa by performing prescribed processing upon the formed image which has been accumulated in the memory.

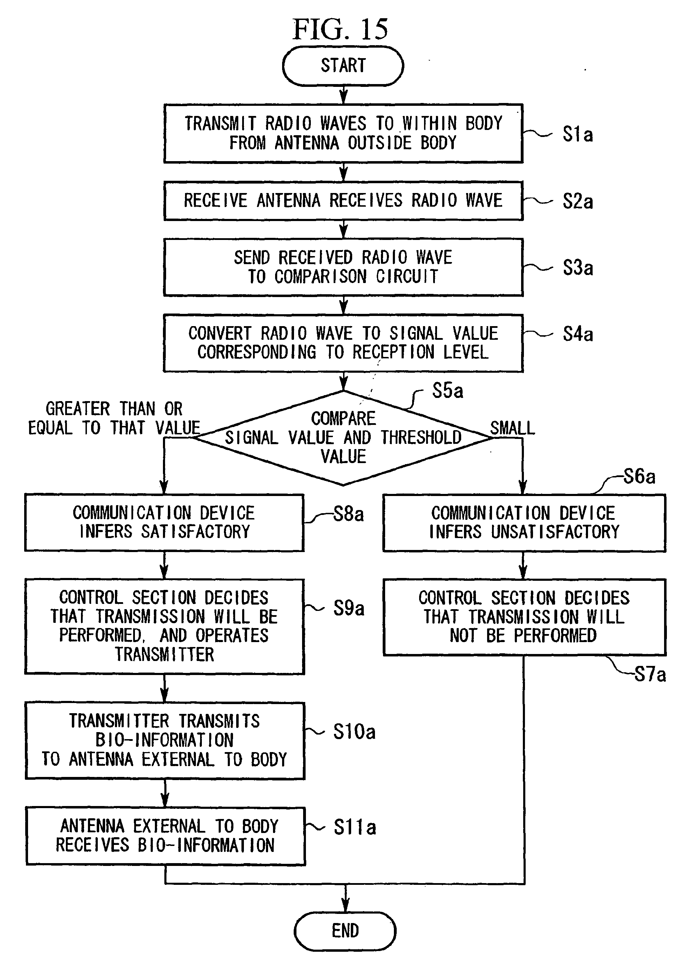

The method of taking the formed image, which is the information for the patient Aa, from the capsule endoscope 110 by communicating from the antennas external to the body of the patient 130 to the capsule endoscope 110 by the capsule endoscope communication system 101 having the above described structure, will now be explained with reference to FIG. 15 etc..

First, as shown in FIG. 9, the patient Aa swallows the capsule endoscope 110 so as to insert it to within his body. While the capsule endoscope 110 which has thus been inserted to within the body of the patient moves within his alimentary canal, along with illuminating the interior of his body with the white colored LEDs 120 which are housed within the vessel 111 as shown in FIG. 11, forms images of the interior of the body of the patient by the image formation element 119 periodically, for example at intervals of a fixed time period. These images which have been formed are subjected to prescribed processing by the processing section 121, and then are stored in the memory 113. In this manner, the capsule endoscope 110 moves within the alimentary canal while acquiring information about the interior of the body of the patient at random, from when it is ingested through his mouth to when it is excreted.

During this period, the images which are formed are sequentially accumulated in the memory 113.

Here, the acquisition of the formed images from the capsule endoscope A is performed after a certain time period has elapsed from when the patient Aa swallows the capsule endoscope 110. In the present example, for example, acquisition of the formed images is performed after the capsule endoscope A has passed to within his stomach. First, as shown in FIG. 9, the patient Aa attaches the air mat 140 to which the antennas external to his body 130 are fitted by winding it around the vicinity of his abdomen. At this time, the air mat 140 is fitted so that a certain gap is left between the air mat 140 and his body. After the fitting, air is injected from an air supply source such as an air pump or the like into the air injection hole 142, and thereby the air mat 140 is tightened about the body of the patient Aa. Then, as shown in FIG. 13, the antennas external to the body of the patient 130 will be in a reliably fixed state, and moreover t be in a state of being separated from the surface of the body of the patient by a prescribed distance ha.

Furthermore, as shown in FIG. 9, the recording device 145 is fitted to the air mat 140 and is connected to the plug 143.

After the air mat 140 has been fitted, upon the operation of a switch not shown in the figures, a radio wave Ba is transmitted from the antennas external to the body of the patient 130 to within his body (the step S1a in FIG. 15). At this time, the radio wave Ba is transmitted simultaneously from the plurality of antennas external to the body of the patient 130. According to its position and its attitude, the signal reception antenna 115 of the capsule endoscope A receives the radio wave Ba from the closest one of the antennas external to the body of the patient 130 (in the step S2a). Furthermore, along with receiving the radio wave Ba, the signal reception antenna 115 transmits the radio wave Ba to the comparison circuit 116 (in the step S3a). The comparison circuit 116, along with converting the radio wave Ba which has been transmitted and which it has received into a signal value according to the level of the received signal (in the step S4a), also compares this signal value and a threshold value which has been set in advance (in the step S5a).

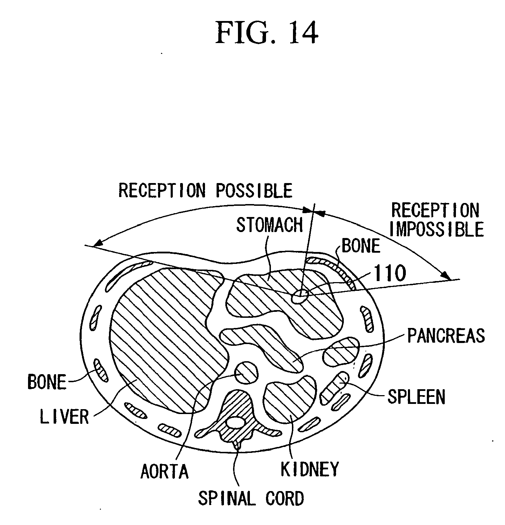

If the result of the comparison is that the value of the signal is smaller than the threshold value, then the comparison circuit 116 infers that the state of communication with the antenna external to the body of the patient 130 is satisfactory (in the step S6a). For example, as shown in FIG. 14, if an internal organ for which the amount of blood flow is great (the liver or the like) or bone or the like is present between the capsule endoscope 110 and the antenna external to the body of the patient 130, which is the radio wave attenuation region, then, since the level of the radio wave Ba which is received is low, it is inferred that the state of communication is not satisfactory. It should be understood that, if the communication state is poor, the radio wave Ba does not reach from the antenna external to the body of the patient 130, and the signal reception antenna 115 does not receive the radio wave Ba.

The comparison circuit 116 transmits the result of the inference that the communication state is not satisfactory to the signal transmission control section 117. The signal transmission control section 117 receives the result, and performs the final decision not to perform communication (in the step S7a). In this case, after the decision, the signal transmission control section 117 does not operate the signal transmission section 114.

On the other hand, if the value of the signal is the same or greater than the value as the threshold value, then the comparison circuit 116 infers that the state of communication with the antenna external to the body of the patient 130 is satisfactory (in the step S8a). In other words, it infers that the state of communication is satisfactory by receiving a radio wave Ba of high output. The comparison circuit 116 transmits the fact that the state of communication is satisfactory to the signal transmission control section 117. The signal transmission control section 117 receives the result, and thereby makes the final decision to perform communication, and then operates the signal transmitter 114a (in the step S9a). Following the decision by the signal transmission control section 117, the signal transmitter 114a transmits the image which has been formed and which is accumulated in the memory 113 as a radio wave Ca to outside the body of the patient via the signal transmission antenna 114b (in the step S 10a).

The antenna external to the body of the patient 130, along with receiving the radio wave Ca which has been transmitted (in the step S11a), also transmits it to the recording device 145 via the plug 143. At this time, since the antennas external to the body of the patient 130 are separated by the prescribed distance ha from his body, any influence of the impedance of the body of the patient can be prevented. Furthermore, it is possible reliably to receive the radio wave Ca which has been transmitted, since it is also possible to receive the radio wave Ca and the like coming from the surroundings. And the recording device 145 records in the memory the formed image, which is the information, which has been transmitted from an antenna external to the body of the patient. By this, it is possible reliably to take out the information from the capsule endoscope 110 which has been ingested into the body of the patient.

According to the capsule endoscope communication system 101 and the capsule endoscope 110, it is possible to obtain the formed images, which are the information, from the capsule endoscope 110, which is shifting within the body of the patient while forming images of various portions thereof, simply by the patient putting on the air mat 140 to which the antennas external to the body of the patient 130 are fitted. In other words, in the capsule endoscope 110, the comparison circuit 116 infers whether or not the state of communication is satisfactory according to the received level of the radio wave Ba which has been transmitted from the antenna external to the body of the patient 130, and then, based upon the result of the inference, the signal transmission control section 117 makes the final decision, and then transmits the images which have been formed to the antenna external to the body of the patient 130 using the signal transmission section 114. By this, it is possible reliably to obtain the information, since the information is received from the capsule endoscope 110 which is shifting within the body of the patient when the state of the communication is satisfactory. In particular, it is possible reliably to obtain outside the body of the patient the information which has been acquired by the capsule endoscope 110 at any time it is required, and this information is not obtained finally all at once, but while the capsule endoscope 110 is shifting within the body of the patient. Therefore, even if, for example, some disappearance or the like of the information has occurred due to some problem with the capsule endoscope 110, it is possible to suppress the influence thereof as minimum, since the information up to the point is already recorded in the recording device 145. Accordingly, it is possible to enhance the reliability of inspection. Furthermore, the capacity of the memory 113 can be saved, and can be utilized with high efficiency. Yet further, as the patient Aa does not need always to wear the air mat 140 which includes the antennas external to his body 130, and as the patient Aa uses it when it is necessary, it is possible to alleviate the burden of the inspection upon the patient Aa.

Furthermore, since the antennas external to the body of the patient 130 are separated by the prescribed distance ha from the body of the patient Aa, it is possible to prevent the influence from the impedance of the patient Aa. Therefore, it is possible reliably to receive the radio wave Ca which is transmitted from the capsule endoscope 110.

### (Second example)

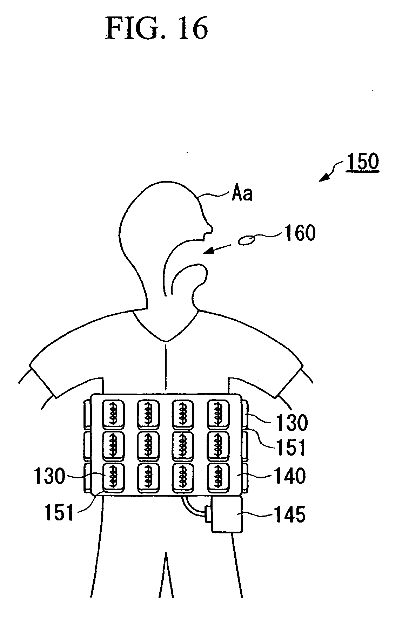

Next, a capsule type medical apparatus according to the second example embodiment of the present will be explained with reference to FIGS. 16 through 18. Moreover, in the explanation of the present example, to structural elements which are the same as in the above described first example the same reference symbols will be affixed, and their explanation will be omitted.

The point of difference between the present example and the above described first example that, whereas in the above described first example the capsule endoscope 110 inferred the state of communication according to the level of the radio wave Ba which had been transmitted from the antenna external to the body of the patient 130, by contrast, with the capsule endoscope communication system (the capsule type medical apparatus communication system) 150 of the present example the capsule endoscope (capsule type medical apparatus) 160 infers the state of communication according to the level of an energy wave Da which has been transmitted from outside the body of the patient.

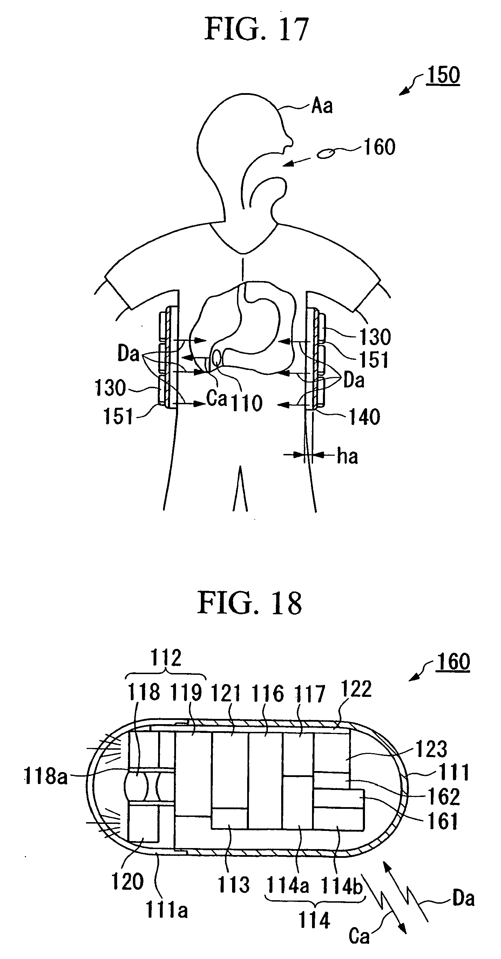

As shown in FIGS. 16 and 17, the capsule endoscope communication system 150 includes: a capsule endoscope 160 which can be swallowed by a patient Aa; antennas external to the body of the patient 130, which are disposed outside the body of the patient, and which receive a radio wave Ca which includes information, in other words, an image which has been formed, from a signal transmission section 114 of the capsule endoscope 160; and an energy wave transmission section (energy wave transmission device) 151 which is arranged adjacent to these antennas external to the body of the patient 130, and which transmits an energy wave Da towards the interior of the body of the patient.

As shown in FIG. 18, the above described capsule endoscope 161 includes an energy wave reception antenna (an energy wave reception device) 160 which receives an energy wave Da which has been transmitted from an energy wave transmission section 151 internal to the vessel 111, and a conversion section 162 which converts the energy wave Da which has been received to electrical power. Furthermore, an energy wave reception antenna 161 is endowed with the function of sending the energy wave Da which has been received to the comparison circuit 116. The comparison circuit 116, along with converting the energy wave Da which has been sent to a signal value which is proportional to the level of the received signal, also compares the signal value with a threshold value which is set in advance, and is set to infer, if the signal value is the same or greater than the threshold value, that the state of communication with the antenna external to the body of the patient 130 is satisfactory.

Furthermore, the signal transmission section 114 is endowed with the function of performing signal transmission by using the electrical power which has been converted from the energy wave Da. In other words, the electrical power which has been converted by the above described conversion section 162 is supplied to the signal transmitter 114a. In other words, the electrical power which the signal transmitter 114a requires for transmission is supplied from outside the body of the patient.

The case of obtaining of information by communicating with the capsule endoscope 160 by the capsule endoscope communication system 150 structured in the above manner will now be explained in the following.

After the capsule endoscope 160 has been introduced to within the body of the patient Aa, along with the patient Aa putting on, according to requirements, the air mat 140 to which a plurality of the antennas external to the body of the patient 130 and the energy wave transmission sections 151 have been fitted, air is injected and a separation from the body of the patient by the prescribed distance ha is established. And an energy wave Da from the energy wave transmission 151 is transmitted to within the body of the patient by a switch or the like not shown in the figure. When the energy wave Da is transmitted, the energy wave reception antenna 161 of the capsule endoscope 160 receives the energy wave Da from that energy wave transmission section 151 which is closest to it, and transmits it to the comparison circuit 116. The comparison circuit 116 compares a signal value which is made to be proportional to the level of the energy wave Da which has been transmitted and received with a threshold value. If the result of the comparison is that the signal value is the same or greater than the threshold value, then the comparison circuit 116 infers that the state of communication with the antenna external to the body of the patient 130 is satisfactory, and sends the result of the inference to the signal transmission control section 117. The signal transmission control section 117 receives the result, and makes the final decision to perform communication and operates the signal transmitter 114a. By this, when the state of communication is satisfactory, the signal transmitter 114a is able to transmit the formed images which are accumulated in the memory 113 to the antenna external to the body 130.

Furthermore, since during the transmission of the signal, the signal transmission is performed by utilizing the electrical power which has been converted by the conversion section 162, it is not necessary to utilize the electrical power of the battery 123 for signal transmission. Since it is possible to take advantage of the electrical power of the battery 123 for acquisition of the information in an intensive manner, it is possible, for example, to increase the number of times for which images are made, or to perform more detailed inspection. Furthermore, even if the service life of the battery 123 has expired, the information which has been acquired up till that time point may reliably be obtained outside the body of the patient.

Furthermore, the signal transmitter 114a may also be set so as to start the signal transmission automatically at the time point that the electrical power which has been converted exceeds the value which is required for driving the signal transmitter 114a.

According to the capsule endoscope communication system 150 and the capsule endoscope 160, it is possible to perform communication when the communication state is inferred as satisfactory based upon the reception level of the energy level Da by the comparison circuit 116, and to obtain the information from within the capsule endoscope 160 which is moving within the body of the patient, at outside the body of the patient in a reliable manner. In particular, since the energy wave Da which has been transmitted from outside the body of the patient is converted into electrical power, and the information is transmitted by utilizing the electrical power, even if the service life of the battery 123 has expired, the information which has been acquired up till that time point may reliably be obtained outside the body of the patient.

It should be understood that the above described energy wave Da may be an electromagnetic wave, or may be an ultrasonic wave.

### (Third example)

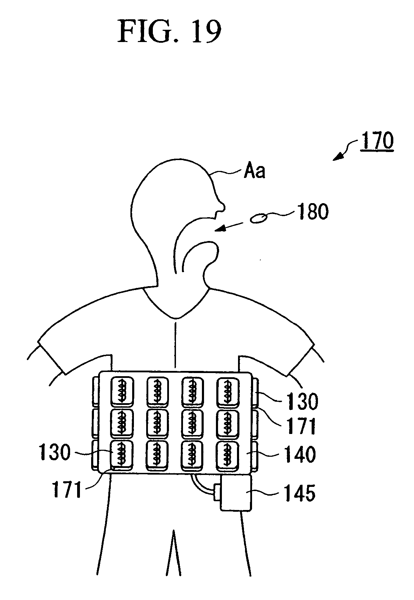

Next, a capsule type medical apparatus according to the third example will be explained with reference to FIGS. 19 through 21. It should be understood that, in the explanation of the present example, to structural elements which are the same as in the above described first example, the same reference symbols will be affixed, and their explanation will be omitted.

The point of difference between the present example and the above described first example that, whereas in the above described first example the capsule endoscope 110 inferred the state of communication according to the level of the radio wave Ba which had been transmitted from the antenna external to the body of the patient 130, by contrast, with the capsule endoscope communication system (the capsule type medical apparatus communication system) 170 of the present example, the capsule endoscope (capsule type medical apparatus) 180 infers the state of communication based upon the magnetic direction of a magnet 171 which is disposed outside the body of the patient.

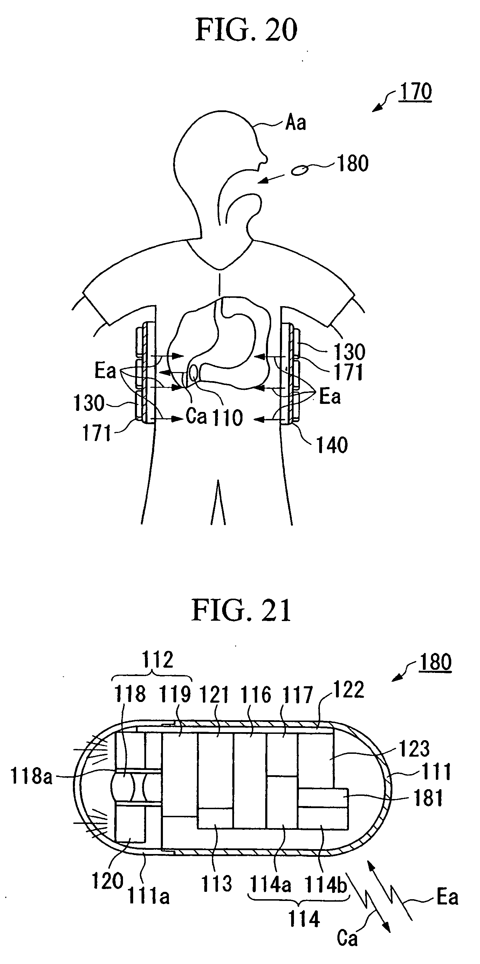

As shown in FIGS. 19 and 20, the capsule endoscope communication system 170 includes: a capsule endoscope 180 which can be swallowed by a patient Aa; an antenna external to the body of the patient 130, which is disposed outside the body of the patient, and which receives a radio wave Ca which includes information, in other words, an image which has been formed, which is transmitted from a signal transmission section 114 of the capsule endoscope 180; and the above described magnet 171 which is disposed in a direction which is correlated with the direction of signal reception (the direction of the plane of polarization) of the antenna external to the body of the patient 130.

As shown in FIG. 21, the above described capsule endoscope 180 includes a magnetic sensor 181 in the interior of the vessel 111, which detects the magnetic direction of the magnet 171 by the magnetic force Ea which the magnet 171 generates. Furthermore, the magnetic sensor 181 is endowed with the function of sending the detected magnetic direction to the comparison circuit 116. Yet further, the direction of the plane of polarization of the signal transmission antenna 114b as seen from the magnetic sensor 181 is set in advance in the comparison circuit 116. By this, by comparing together the magnetic direction which has been sent from the magnetic sensor 181 and the direction which was set in advance, the comparison circuit 116 is able to infer whether or not the direction of signal transmission (the direction of the plane of polarization) of the signal transmission antenna 114b is oriented in the direction of the magnet 171. In other words, the comparison circuit 116 is able to infer whether or not the signal transmission direction of the signal transmission antenna 114b is oriented in the signal reception direction of the antenna external to the body of the patient, since a correlation is established between the magnet 171 and the signal reception direction of the antenna external to the body of the patient 130.

The case of obtaining a information by the capsule endoscope communication system 170 of the above structure by communicating with the capsule endoscope 180 will now be explained in the following.

With this capsule endoscope 180 which is inserted within the body of the patient and which is shifting about while acquiring information via the image formation section 112, when the state of communication is satisfactory and moreover it has shifted to the magnetic field region of the magnet 171, along with the magnetic sensor 181 detecting the magnetic direction by the reaction of the magnet 171 upon the magnetic force Ea, this is also sent to the comparison circuit 116. The comparison circuit 116 compares the magnetic direction which has been sent and has arrived with the direction which is set in advance.

If the result of the comparison is that the magnetic direction is the same direction as the set direction, then it is inferred that the communication state is satisfactory with the matching between the signal transmission antenna 114b and the antenna external to the body of the patient 130 by being most suitable. Accordingly, it is possible reliably to transmit the information, which is the images which have been formed and which are accumulated in the memory 113, to the antenna external to the body of the patient 130.

Since, according to the capsule endoscope communication system 170 and the capsule endoscope 180, the comparison circuit 116 compares together the magnetic direction which has been detected by the magnetic sensor 181 and the direction which has been set in advance, and infers the state of communication by the matching between the communication antenna 114b and the antenna external to the body of the patient 130, it is possible to perform signal transmission only when the communication state is satisfactory, and it is possible reliably to obtain the information outside the body of the patient.

### (Fourth example)

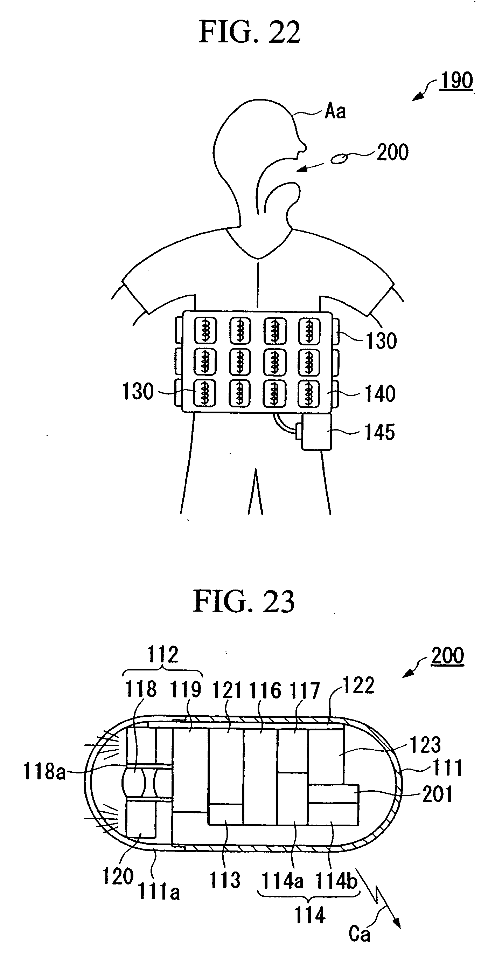

Next, a capsule type medical apparatus according to the fourth example of the present invention will be explained with reference to FIGS. 22 and 23. It should be understood that, in the explanation of the present example, to structural elements which are the same as in the above described first example, embodiment, the same reference symbols will be affixed, and their explanation will be omitted.

The point of difference between the present example and the above described first example is that, whereas in the above described first example, the capsule endoscope 110 inferred the state of communication according to the level of the radio wave Ba which had been transmitted from the antenna external to the body of the patient 130, by contrast, with the capsule endoscope communication system (the capsule type medical apparatus communication system) 190 of the present example, the capsule endoscope (capsule type medical apparatus) 200 infers the state of communication based upon the gravity direction which is detected by a gravity sensor 201.

As shown in FIG. 22, the capsule endoscope communication system 190 of the present example includes a capsule endoscope 200 which can be swallowed by a patient Aa, and an antenna external to the body of the patient 130, which is disposed outside the body of the patient, and which receives a radio wave Ca which includes information, in other words an image which has been formed, from a signal transmission section 114 of the capsule endoscope 200. It should be understood that, in the present example, when the patient Aa has put on the air mat 140, the signal reception direction of the antenna external to the body of the patient 130 is oriented along the direction of gravity.

As shown in FIG. 23, the above described capsule endoscope 200 includes, internally to the vessel 111, the above described gravity sensor 201 which detects the direction of gravity. The gravity sensor 201 is endowed with the function of sending the direction of gravity which it has detected to the comparison circuit 116. The direction of the plane of polarization of the signal transmission antenna 114b as seen from the gravity sensor 201 is set in advance in the comparison circuit 116. By this, the comparison circuit 116 is able to infer whether or not the direction of signal transmission (the direction of the plane of polarization) of the signal transmission antenna 114b is oriented in the signal reception direction of the antenna external to the body of the patient 130 by comparing together the direction of gravity which has been sent by the gravity sensor 201 and the direction which is set in advance.

According to the capsule endoscope communication system 190 and the capsule endoscope 200 which are configured like this, the capsule endoscope 200 which is inserted within the body of the patient and which is shifting about while acquiring information with the image formation section 112, always detects the direction of gravity by the gravity sensor 201 without any relationship to its own attitude. The comparison circuit 116 compares together the direction of gravity which has been sent from the gravity sensor 201 and the direction which has been set in advance. If the result of the comparison is that the direction of gravity is the same direction as the set direction, then it infers that the state of matching of the signal transmission direction of the signal transmission antenna 114b and the signal reception direction of the antenna external to the body of the patient 130 is the most suitable one, in other words, that the communication state is satisfactory. Accordingly, it is possible reliably to transmit the information, which is the formed images which have been accumulated in the memory 113, to the antenna external to the body of the patient 130.

It should be understood that, in the present example, when obtaining the information of the patient Aa from the capsule endoscope 200, it is desirable for him to sit, for example, in a chair or the like, so that the plane of polarization of the antenna external to the body of the patient 130 is stationary and so that it is reliably oriented in the direction of gravity.

Furthermore, a structure would also be acceptable in which the antenna external to the body of the patient 130 moves passively or actively, so that, even if the patient Aa has moved around after having put on the air mat 140, the signal reception direction of the antenna external to the body of the patient 130 is always oriented in the direction of gravity.

Yet further, although the attitude of the capsule endoscope 200 within the body of the patient has been detected by the gravity sensor 201, this is not to be construed as being limitative; for example, instead of the gravity sensor 201, it would also be acceptable to incorporate a giro. In this case, since it is possible to detect a permanently constant direction without any relationship with the attitude within the body of the patient, it is possible to infer the state of communication by the comparison circuit 116 making a comparison with the set direction.

### (Fifth example)

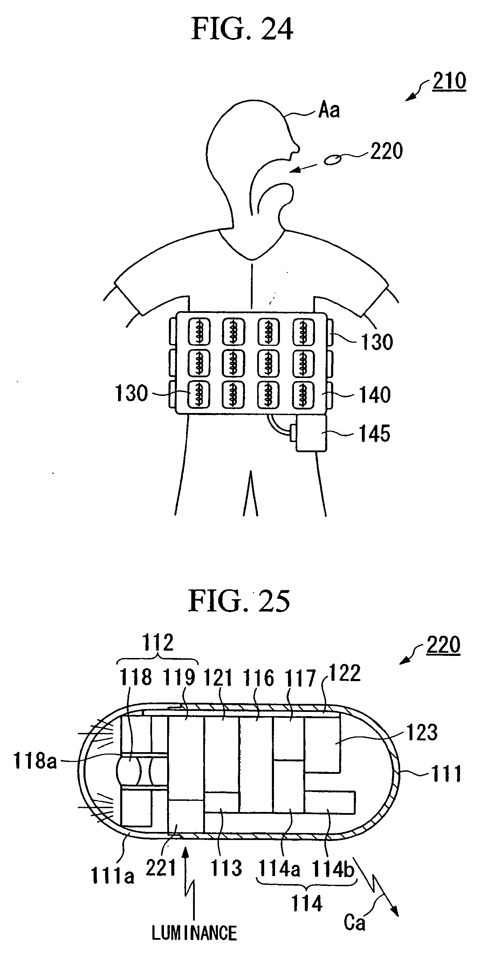

Next, a capsule type medical apparatus according to the fifth example will be explained with reference to FIGS. 24 and 25. It should be understood that, in the explanation of the present example, to structural elements which are the same as in the above described first example, the same reference symbols will be affixed, and their explanation will be omitted.

The point of difference between the present example and the above described first example is that, whereas in the above described first example the capsule endoscope 110 inferred the state of communication according to the level of the radio wave Ba which had been transmitted from the antenna external to the body of the patient 130, by contrast, with the capsule endoscope communication system (the capsule type medical apparatus communication system) 210 of the present example, the capsule endoscope (capsule type medical apparatus) 220 infers the state of communication based upon change of the luminance which is detected by a luminance sensor 221.

That is, as shown in FIG. 24, the capsule endoscope communication system 220 of the present example includes the above described capsule endoscope 220 which can be swallowed by a patient Aa, and an antenna external to the body of the patient 130, which is disposed outside the body of the patient, and which receives a radio wave Ca which includes information, in other words an image which has been formed, from a signal transmission section 114 of the capsule endoscope 220.

As shown in FIG. 25, the above described capsule endoscope 220 includes, in the interior of the vessel 111, the luminance sensor 221 which detects the luminance within the body of the patient. A portion of the luminance sensor 221 is exposed at the outer surface of the vessel 111, and is accordingly enabled to detect the luminance within the body of the patient. Furthermore, the luminance sensor 221 is endowed with the function of sending the detected luminance to a comparison circuit 116. Yet further, for example, a luminance value corresponding to the unique value of a stomach where the communication state is a comparatively satisfactory state, is set in advance in the comparison circuit 116 as a threshold value. By doing this, the comparison circuit 116 is able to infer that the capsule endoscope 220 is positioned in the stomach, that is, the state of communication is in satisfactory by comparing the luminance value which has been sent by the luminance sensor 221 with the threshold value.

According to the capsule endoscope communication system 210 and the capsule endoscope 220 having the above configuration, the capsule endoscope 220 which is inserted into the body of the patient and which is shifting around while acquiring information via the image formation section 112, the luminance sensor 221 always detects the luminance within the body of the patient. Here, if the capsule endoscope 220 has shifted within the body of the patient, then the comparison circuit 116 compares together the luminance value which has been sent from the luminance sensor and the threshold value, and infers that it is currently positioned within the stomach. Accordingly, the information, which is the images which have been formed and which are accumulated in the memory 113, can be reliably transmitted to the antenna external to the body of the patient 130, since it is possible to perform communication when the capsule endoscope 220 is positioned within the stomach where the communication state is comparatively satisfactory.

It should be understood that although, in the present embodiment, it is inferred that the capsule endoscope 200 is positioned within the stomach where the communication state is satisfactory according to the luminance value which has been detected by the luminance sensor 221, this is not to be construed as being limitative; for example, it would also be acceptable to use a pH sensor instead of the luminance sensor 221. In this case, it would be possible to infer whether or not the capsule endoscope 210 is positioned within the stomach by setting in advance a pH value which is unique value of the stomach in the comparison circuit 116 as a threshold value. Or, alternatively, it would also be acceptable to employ, as a reference for inference, not a threshold value of luminance or of pH value, but rather a pattern of change of luminance or of pH value up to the desired position.

Furthermore although, in the present example, the position where the state of communication was comparatively satisfactory was set to the stomach of the patient, this is not to be construed as being limitative.

### (Sixth example)

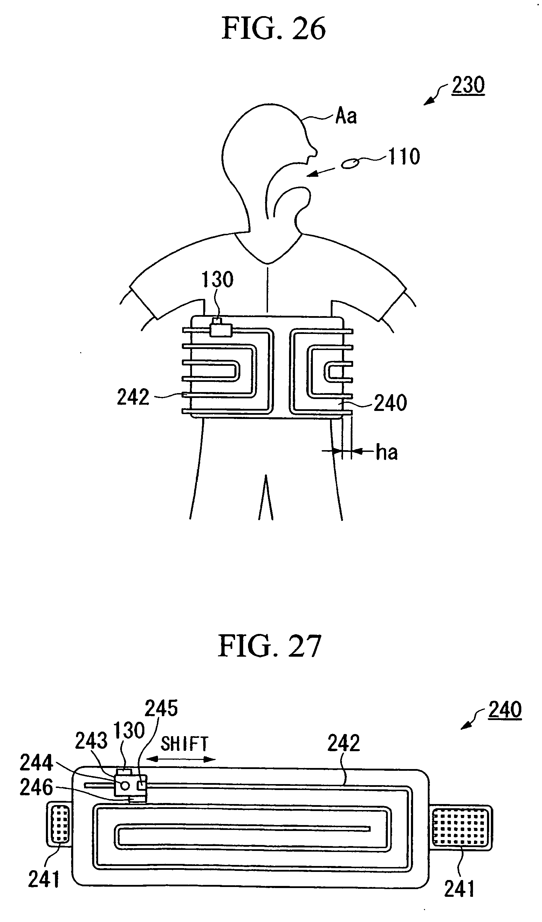

Next, a capsule type medical apparatus according to the sixth example will be explained with reference to FIGS. 26 through 28. It should be understood that, in the explanation of the present example, to structural elements which are the same as in the above described first example, the same reference symbols will be affixed, and their explanation will be omitted.

The point of difference between the present example and the above described first example is that, whereas in the above described first example communication with the capsule endoscope 110 was performed by the patient Aa wearing the air mat 140 to which the plurality of antennas external to the body of the patient 130 were fixed, by contrast, with the capsule endoscope communication system (the capsule type medical apparatus communication system) 230 of the present the antenna external to the body 130 is movable.

As shown in FIG. 26, the capsule endoscope communication system 230 of the present example includes a capsule endoscope 110 which can be swallowed by a patient Aa, and an air mat 240 which includes the above described antennas external to the body of the patient 130.

As shown in FIG. 27, the air mat 240 is formed in a belt shape, and a connector 241 such as Velcro fasteners or the like are provided on the front and rear surfaces of the both ends. By this, it is possible for the patient Aa to keep it rolled upon, for example, the vicinity of his abdomen. Furthermore, a shift rail 242 which has a height of ha is provided upon the outer surface of the air mat 240, over its entire surface. Yet further, a shift pedestal 243 which can be shifted manually is attached upon the shift rail 242, and the above antenna external to the body of the patient 130 is attached upon this shift pedestal 243.

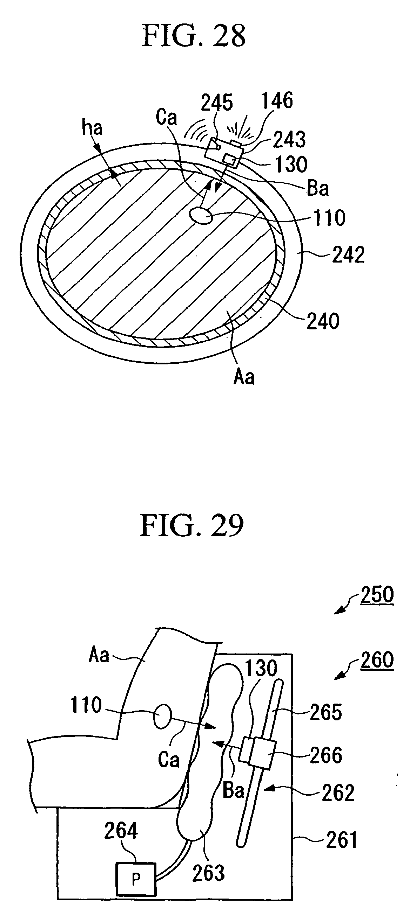

In other words, as shown in FIG. 28, the antenna external to the body of the patient 130, along with being in a state of being separated from the surface of the body of the patient Aa by the prescribed interval distance ha, can also be shifted in the state keeping the interval distance ha.

Furthermore, to the shift pedestal 243, there are provided a recording device 244 which receives the information which has been received by the antenna external to the body of the patient 130, a speaker 245 which operates when the antenna external to the body of the patient 130 has received the radio wave Ca, and a LED 146 which emits light when the antenna external to the body of the patient 130 has received the radio wave Ca.

Whenever required, the above described recording device 244 accumulates images of the patient Aa which have been formed and which have been sent from the antenna external to the body of the patient 130 in a memory not shown in the figures. The above described speaker 245, for example, may be set so as to generate a continuous sound when the antenna external to the body of the patient 130 is receiving the radio wave Ca, and so as to generate an intermittent sound when signal reception has been terminated. Furthermore, the LED 146, for example, may be set so as to be illuminated when the antenna external to the body of the patient 130 is receiving the radio wave Ca, and so as to be turned off when signal reception has been terminated.

When taking the information by communicating with the capsule endoscope 110 using the capsule endoscope communication system 230 which has the abovementioned structure, the patient Aa, after having put on the air mat 240, slowly shifts the shift pedestal 243 along the shift rail 242 by manual operation. Meanwhile, the capsule endoscope 110 infers the state of communication based upon the received level of the signal of the radio wave Ba which is transmitted from the antenna external to the body of the patient 130. Here if, by shifting the antenna external to the body of the patient 130, when it has been inferred that the state of communication is satisfactory, then the capsule endoscope 110 transmits the radio wave signal Ca towards the antenna external to the body of the patient 130. When the antenna external to the body of the patient 130 receives the radio wave Ca, along with sending the information to the recording device 244, it notifies the speaker 245 and the LED 146. Upon receipt of the notification, the speaker 245 generates a continuous sound, while the LED 146 turns ON. Here, since the patient Aa notices that the radio wave Ca has been received by the antenna external to the body of the patient 130 according to the sound from the speaker 245 and the light generated by the LED 146, therefore he stops shifting the shift pedestal 243. By this, it is possible reliably to obtain the information for the patient Aa by the single antenna external to the body of the patient 130 when the state of communication from the capsule endoscope 110 is satisfactory. Furthermore since, when the reception of the information has been completed, along with the speaker 245 generating an intermittent sound, also the LED 146 is turned OFF, accordingly the patient Aa is easily enabled to know that the reception of signals has been completed.

Since, as has been described above, according to the capsule endoscope communication system 230, a position in which the state of communication is satisfactory may be searched for over a wide range by shifting the antenna external to the body of the patient 130 along the shift rail 242, accordingly, it is possible to utilize a single antenna external to the body of the patient 130 in an efficient manner.

It should be understood that, in the present example it is possible easily to change the distance between the antenna external to the body of the patient 130 and the body of the patient Aa itself by changing the height ha of the shift rails. Furthermore although, in order to cause the patient Aa to perceived that the radio wave Ca has been received, a construction was employed in which both the speaker 245 and the LED 146 were provided, it would also be acceptable to provide only one of them; and it would also be acceptable to provide a different perception device. Yet further, although the shift pedestal 243 was moved manually, it would also be acceptable for it to be shifted electrically by a motor or the like.

### (Seventh example)

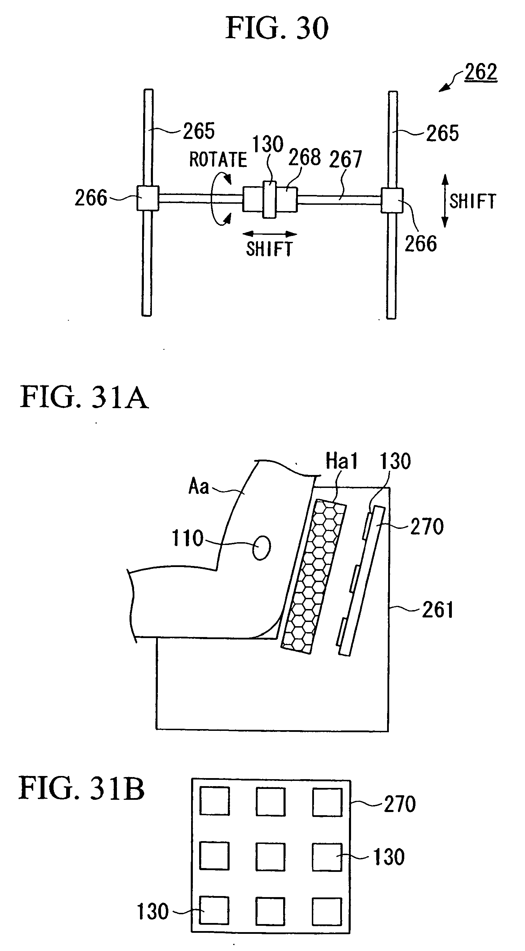

Next, a capsule type medical apparatus according to the seventh example will be explained with reference to FIGS. 29 and 30. It should be understood that, in the explanation of the present example, to structural elements which are the same as in the above described first example, the same reference symbols will be affixed, and their explanation will be omitted.

The point of difference between the present example and the above described first example is that, whereas in the above described first example, communication with the capsule endoscope 110 was performed by the patient Aa wearing the air mat 140 to which the plurality of antennas external to the body of the patient 130 were fixed, by contrast, with the capsule endoscope communication system (the capsule type medical apparatus communication system) 230 of the present example, communication with the capsule endoscope 110 is performed by a communication device 260 which includes an antenna external to the body of the patient 130, which is disposed, for example, in a hospital or the like.

As shown in FIGS. 29 and 30, the capsule endoscope communication system 250 of the present example includes a capsule endoscope 110 which can be swallowed by a patient Aa, and the above described communication device 260.

The communication device 260 internally houses, within the chair 261, the above described antenna external to the body of the patient 130, a shifting mechanism 262 which changes the position and the orientation of this antenna external to the body of the patient 130, an air bag 263 which adjusts the distance between the antenna external to the body of the patient 130 and the patient Aa, and an electrically driven pump 264 which supplies air to within the air bag 263.

The above described shifting mechanism 262 includes a pair of parallel vertical guides 265 at the backrest of the chair 261, a pair of vertical sliders 266 which can be shifted in the axial direction of these vertical guides 265, a horizontal guide 267 which is connected between the pair of vertical sliders 266, and a horizontal slider 268 which, along with being shiftable in the axial direction of this horizontal guide 267, also can be rotated around the axis of this horizontal guide 267. And the above described antenna external to the body of the patient 130 is attached to the horizontal slider 268. By this, the antenna external to the body of the patient 130 can be shifted in the upward and downward direction over the entire extent of the backrest of the chair 261, while its angle is varied.

It should be understood that the vertical slider 266 and the horizontal slider 268 are shifted by motors not shown in the figures.

The above described air bag 263 is arranged between the shifting mechanism 262 and the backrest of the chair 261, and, when air is supplied from an electrically driven pump 264, it expands and blows up the backrest. In other words, when the patient Aa has sat down in the chair 261, the distance between the patient Aa and the antenna external to the body of the patient 130 is spaced to the prescribed interval distance.

When taking out information by communicating with the capsule endoscope 110 by the capsule endoscope communication system 250, after having swallowed the capsule endoscope 110, the patient Aa sits in the chair 261 which is installed in a hospital or the like. When he sits in the chair 261, the antenna external to the body of the patient 130 shifts by shifting of the vertical sliders 266 and by shifting and rotating of the horizontal sliders 268. Meanwhile, the capsule endoscope 110 infers the state of communication based upon the level of signal reception of the radio wave Ba which has been transmitted from the antenna external to the body of the patient 130. Here, if it has been inferred that the state of communication is satisfactory by shifting the antenna external to the body of the patient 130, then the capsule endoscope 110 transmits the radio wave Ca towards the antenna external to the body of the patient 130.

In this manner, the patient Aa can obtain the information by communicating with the capsule endoscope 110, merely by sitting in the chair 261, and by searching for a position in which the state of communication is satisfactory while shifting the antenna external to the body of the patient 130 within the range of the backrest. Accordingly, it is possible to alleviate the burden upon the patient Aa due to the acquisition of the information. Furthermore, it is possible reliably to diminish the influence of impedance, since it is possible to secure the distance between the antenna external to the body of the patient 130 and the patient Aa easily and reliably by the air bag 263, corresponding to the physique of the patient Aa.

It should be understood that it would be acceptable to inform the patient Aa that the radio wave Ca is being received in a position in which the state of communication with the antenna external to the body of the patient 130 is satisfactory with, for example, a sound from a speaker, or a light from a LED, or the like.

Yet further, although the antenna external to the body of the patient 130 was made movable by the shifting mechanism 262, this is not to be construed as being limitative; it would also be acceptable to arrange a plurality of such antennas as being fixed.

For example, as shown in FIGS. 31A and 31B, it would be acceptable to provide a plate shaped antenna array 270 within the backrest of the chair 261, and to arrange a plurality of antennas external to the body of the patient 130 in the antenna array 270. In this case, the closest one to the capsule endoscope 110 of the antennas external to the body of the patient 130 receives the radio wave Ca when the state of communication is satisfactory. Furthermore, it would also be acceptable to utilize a foamed member Hal which was made from foamed styrol or the like, in order to secure the distance between the antenna external to the body of the patient 130 and the patient Aa.

It should be understood that the technology of the present invention is not one which is limited to the above described embodiments; it is possible to add various changes.

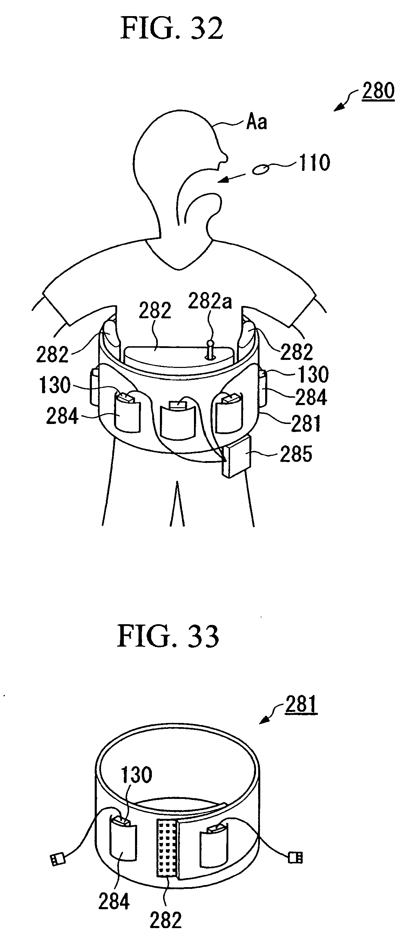

In the above described first example, although an air mat into which air could be injected was utilized for keeping the antenna external to the body of the patient at the prescribed distance from the body of the patient, this is not to be construed as being limitative. For example, the endoscope communication system (the capsule type medical apparatus communication system) 280 shown in FIG. 32 includes a mat 281 and an air bag 282 which can be put on by the patient Aa. As shown in FIG. 33, the mat 281 is made in a belt shape, and a connector 282 such as Velcro fasteners or the like are provided at both ends of its front and rear surfaces. By this, it can be fitted by winding it around the patient Aa. Furthermore, upon the outer surface of the mat 281, there are formed a plurality of pockets 284 in which antennas external to the body of the patient 130 can be stored. Yet further, cables are connected via plugs to the antennas external to the body of the patient 130.

The above described air bag 282 has roughly the same height as the above described mat 281, and is made so that air can be injected into its interior from an air injection hole 282a.

With this endoscope communication system 280, when communicating with the capsule endoscope 110, the patient Aa, as shown in FIG. 32, puts on the mat 281 while sandwiching the air bag 282 between it and himself. After putting it on, along with storing the antennas external to the body of the patient 130 in each pocket 284, the plugs of the antennas external to the body of the patient 130 is connected to receptacles of a recording device 285. By doing this, it is possible to affix a plurality of antennas external to the body of the patient 130 at a prescribed distance from the body of the patient.

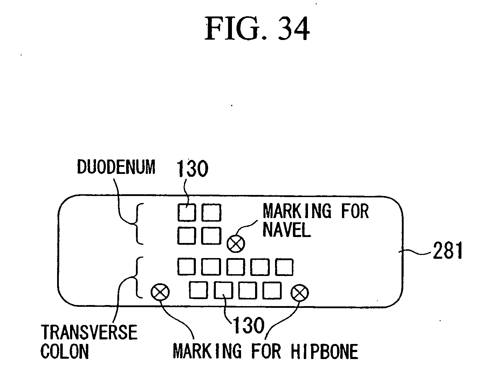

Furthermore, as shown in FIG. 34, it would also be acceptable to append markings to the above described mat 281 for, for example, the navel or the hip bone. Yet further, the pockets 284 are formed centered on each marking. In this case, by putting on the mat 281 while using the markings as guides, it is possible for the patient Aa to position the antenna external to the body of the patient 130 in an intensive manner to a specific region, such as, for example, the vicinity of the duodenum, the vicinity of the transverse colon, or the like. Accordingly, when the capsule endoscope 110 has moved to the vicinity of the duodenum or the vicinity of the transverse colon, it is possible to perform communication with it. However, this process is not limited to the vicinity of the duodenum or the vicinity of the transverse colon.

Furthermore although, in the above described embodiments, formed images of various portions within the body of the patient and these images were taken as the information, this is not to be construed as being limitative; it would also be acceptable for the information to be pH value, pressure, or body fluid or the like. In this case, it would be appropriate, instead of providing an image formation section, to adopt the structure which can acquire such various information.

Yet further, although a device was employed for the image formation section which took photographs within the body of the patient at fixed time intervals intermittently and moreover at random, this is not to be construed as being limitative; it would also be acceptable, for example, to utilize a device which takes photographs of the interior of the body of the patient continuously, such as a video or the like. In this case, the video signal would be stored.

Yet further, the present invention is not to be construed as being limited to the case of photography within the body of the patient by video or the like; any device which is able to detect information within the body of the patient and to transmit the resulting data to a device outside the body of the patient will be acceptable. For example, the present invention may also be applied to a capsule type medical apparatus for hemorrhage inspection which includes a hemoglobin sensor, or to a capsule type medical apparatus for inspection of information within the living body which acquires information relating to pH value, pressure value, temperature, amount of bacteria, genetic abnormality, and the like within the body of the patient intermittently over a long time period and transmits that information to a device outside the body of the patient, or to an ultrasonic wave capsule type medical apparatus which acquires ultrasonic wave images or the like intermittently and transmits them to a device outside the body of the patient.

Even further, it would be acceptable to incorporate a memory which was equipped with a backup function to the capsule endoscope, and to store up all the images which are formed, including the formed images which have been transmitted to outside the body of the patient by the signal transmission section. In this case, it would be possible to enhance the reliability of inspection, since it would be possible to collect the image formation data from the memory after receiving the capsule endoscope. Moreover, even if, when transmitting the data for the images which have been formed to outside the body of the patient, the signal were to be interrupted during the transmission, it would also be possible, during the next signal transmission, to transmit the formed images from after the break point.

Although preferred embodiments of the present invention have been explained above, the present invention is not limited to these embodiments. . The present invention is not limited by the above described explanations, but is limited only by the scope of the appended Claims.

As explained above, the first capsule type medical apparatus communication system of the present invention includes a capsule type medical apparatus which transmits information relating to the body of an examinee from within the body of the examinee to outside it, and an information receiver which is disposed externally to the body of the examinee and which receives the information, and: the capsule type medical apparatus transmits towards the information receiver a communication confirmation signal which confirms the state of communication with the information receiver; the information receiver, when it has received the communication confirmation signal, transmits a communication authorization signal which authorizes the capsule type medical apparatus to perform communication; and the capsule type medical apparatus includes a communication control device which transmits the information when it has received the communication authorization signal.

With the above described first capsule type medical communication system, since the capsule type medical apparatus transmits the information when it has received the communication authorization signal which has been transmitted from the information receiver, accordingly, it is possible to perform the transmission of the information (the data signal transmission) when the state of communication between both of them is satisfactory. Accordingly, when the state of communication is unsatisfactory, i.e. when the information receiver cannot receive the information, then there is no undesirable consumption of electrical power for transmitting useless images from the capsule type medical apparatus. Furthermore, since the information such as, for example, a plurality of image frames or the like is transmitted when the state of communication is satisfactory, the possibility that the information receiver loses the images which have been acquired is small, and the information is received in an efficient manner. Thus, it is possible to obtain more accurate information.

The communication authorization signal may also serve as a wireless signal which performs supply of electrical power to the capsule type medical apparatus.

In this case, since the capsule type medical apparatus is able to receive supply of electrical power via the communication authorization signal from the information receiver which is disposed outside the body of the examinee, it is possible to eliminate cutoff of electrical power due to the battery becoming exhausted or the like. Thus, it is possible to obtain the information in a reliable manner.

The first capsule type medical apparatus of the present invention includes: an acquisition device which acquires information about the body of an examinee; a signal transmission device which transmits, towards an information receiver which is disposed externally to the body of the examinee, the information which has been acquired, and a communication confirmation signal which confirms the state of communication with the information receiver; a signal reception device which, if the communication confirmation signal has been received, receives a wireless signal which has been transmitted from the information receiver which includes at least a communication authorization signal; and a communication control device which makes a decision as to whether or not to transmit the information, based upon the state of reception of the communication authorization signal.

When the above described first capsule type medical apparatus is inserted within the body of the patient who is to be examined, it moves within the body of the patient who is to be examined (due to peristaltic movement and the like) while it is performing the acquisition of the information with the acquisition device. Furthermore, when it shifts within the body of the patient who is to be examined, at the same time as acquiring the information, the signal transmission device transmits the communication confirmation signal to outside the body of the examinee. When the information receiver receives the communication confirmation signal, it transmits a wireless signal which includes the communication authorization signal. And, when the signal reception device receives the communication authorization signal which has been sent from the information receiver when it is shifting within the body of the patient who is being examined, the communication control device makes a decision as to whether or not to transmit the information, based upon the state of signal reception of the communication authorization signal, and, if it decides to transmit, it transmits the information from the signal transmission device.

In this manner, it is possible to transmit the information (data transmission) when the state of communication between the first capsule type medical apparatus and the information receiver is satisfactory. Accordingly there is no undesirable consumption of electrical power for transmitting useless images when the state of communication is unsatisfactory, in other words, when the information receiver cannot receive the information. Furthermore, since the information such as, for example, a plurality of image frames or the like is transmitted when the state of communication is satisfactory, the possibility that the information receiver loses the images which have been acquired is small. Thus, it is possible to obtain more accurate information.

The same antenna may be used for both the signal transmission device and the signal reception device.

In this case, since the signal transmission device and the signal reception device can employ the same antenna by changing it over or the like, it is possible to anticipate an enhancement of compactness.

The signal reception device may include an envelope wave detection circuit.

In this case, it is possible to limit the consumption of electrical power by the signal reception device, since it is possible to manufacture the main structural components of the signal reception device such as, for example, the greater portion of the demodulation portion and the like, as passive components. In particular, if a battery or the like which is housed internally within the first capsule type medical apparatus is utilized as the electrical power source, it is possible to reduce the consumption of the electrical power of the battery, and accordingly it is possible to extend its service life, since it is possible efficiently to employ its limited electrical power.

The signal reception device may include a rectification circuit which obtains electrical power from the wireless signal, and a communication authorization detection section which detects the communication authorization signal from the output of the rectification circuit and sends it to the communication control device.

In this case, it is possible to eliminate cutoff of electrical power due to the battery becoming exhausted or the like, since it is possible for the rectification circuit to receive the supply of electrical power via the wireless signal which is transmitted from the information receiver which is arranged externally to the body of the examinee. Accordingly, it is possible to obtain the information in a reliable manner. Furthermore, it is not necessary to provide any separate detection circuit, since the communication authorization detection section detects the communication authorization signal from the output of the rectification circuit. Accordingly, the construction is easy, and it is possible to anticipate an enhancement of the compactness.

The information receiver of the present invention includes: a signal reception device which receives, from a capsule type medical apparatus within the body of an examinee, information about the body of the examinee which has been transmitted, and a communication confirmation signal which confirms the state of communication with the capsule type medical apparatus; a recording section which records the information which has been received; a communication authorization signal creation section which, when the signal reception device has received the communication confirmation signal, creates a communication authorization signal which authorizes the capsule type medical apparatus to transmit the information; and a communication authorization signal transmission device which transmits the communication authorization signal.

With the above described information receiver, when the communication confirmation signal is received by the signal reception device from the capsule type medical apparatus within the body of the patient who is being examined, the communication authorization signal creation section creates the communication authorization signal which authorizes the transmission of the information. In other words, by receiving the communication confirmation signal, the communication authorization signal creation section decides that the state of communication with the capsule type medical apparatus is satisfactory, and performs creation of the communication authorization signal. And the communication authorization signal transmission device transmits the communication authorization signal to the capsule type medical apparatus. The capsule type medical apparatus transmits the information when the communication authorization signal is received. The information is received by the signal reception device, and then is also recorded in the recording section.

In this manner, the communication authorization signal is transmitted to the capsule type medical apparatus when, based upon the communication confirmation signal, the state of communication between the capsule type medical apparatus and the information receiver is satisfactory, so that it is possible to obtain the information. Furthermore, since the information such as, for example, a plurality of image frames or the like is obtained when the state of communication is satisfactory, the possibility that the images which have been acquired be lost is small. Thus, it is possible to obtain more accurate information.

The communication authorization signal may also serve as a wireless signal which performs supply of electrical power to the capsule type medical apparatus.

In this case, since it is possible to utilize the wireless signal for supplying electrical power and the communication authorization signal together, it is possible to perform the supply of electrical power and the transmission of the communication authorization signal by the same signal transmission device. Therefore, the construction can be simple, and it is possible to enhance the compactness.

When the communication confirmation signal is not received, the communication authorization signal transmission device may transmit the communication authorization signal over an interval which is longer than the signal transmission interval of the communication confirmation signal.

In this case, since the communication authorization signal which also serves for supply of electrical power is transmitted, and thus the electrical power is supplied in a timely manner to the capsule type medical apparatus, over a interval which is longer than the interval over which the communication confirmation signal is transmitted, it is possible to prevent failure of transmission of the communication confirmation signal due to cutoff of the electrical power of the capsule type medical apparatus. Therefore, it is possible to obtain the information in a reliable manner.

Accordingly, according to the first capsule type medical apparatus communication system, the first capsule type medical apparatus, and the information receiver of the present invention which have been explained above, it becomes possible to perform the transmission and/or the reception of the information when the state of communication between the capsule type medical apparatus and the information receiver is satisfactory.

Furthermore, when the state of communication is unsatisfactory, in other words, when the information receiver is not able to receive the information, there is no consumption of electrical power due to transmission of useless images from the capsule type medical apparatus. Moreover, since the information such as, for example, a plurality of image frames or the like, is transmitted when the state of communication is satisfactory, loss by the information receiver of the images which have been acquired is kept small, and the information is received in an efficient manner. Therefore, it is possible to obtain the information in a more accurate manner.

The second capsule type medical apparatus (which is termed the second capsule type medical apparatus in order to distinguish it from the first capsule type medical apparatus) includes, in a container which is introduced to within the living body: an acquisition device which acquires information about the interior of a living body; a signal transmission device which transmits the information which has been acquired by the acquisition device to outside the living body; a sensor which detects information which specifies the position and/or the attitude of the container within the living body; an inference device which infers the state of communication with outside the living body based upon the information which has been detected by the sensor; and a signal transmission control device which controls the signal transmission device, based upon the results of inference by the inference device.

With the above described second capsule type medical apparatus, after it has been introduced to within the living body or the like, the information is acquired by the acquisition device while it is shifting around within the living body. Furthermore, since its own position and/or attitude are specified at the same time as the information is being acquired, the information such as the pH value or the like within the living body or the information of the radio wave or the like from outside the living body is detected and is sent to the inference device. The inference device infers the state of communication with the exterior of the living body based upon the information which has been sent and has arrived. For example, if the information which has been sent and has arrived from the sensor is a radio wave from outside the living body, then, by comparing the magnitude of the radio wave when it has been received with a threshold value which has been set in advance, it is possible to infer whether or not it is possible to communicate with outside the living body. The signal transmission control device finally decides upon the state of communication with outside of the living body, based upon the results of inference by this type of inference device, and, if it has been decided that it is possible to perform communication, it operates the signal transmission device and transmits the information towards the exterior of the living body.

While shifting about within the living body, the information which has been acquired is transmitted to the outside of the living body only when the state of communication with the outside of the living body is satisfactory. Accordingly, it is possible to transmit the information about the patient or the like reliably to outside the living body. Furthermore, since the information which has been acquired is not transmitted all at once at the end of acquisition, but is transmitted to outside the living body at any time when the position ensures a satisfactory state of communication, accordingly, if a memory is provided for storing the information, it is possible to keep the capacity of this memory low, and it is possible to utilize the memory with good efficiency.

As the sensor, a magnetic sensor which detects magnetic direction may be employed.

In this case, it is possible to detect the magnetic direction by the magnetic sensor from the magnetism of the earth, or from a magnet which is disposed outside the living body, or the like. In other words, it is possible for the sensor always to detect the same direction within the living body, irrespective of its own attitude. By this, it is possible for the inference device to infer whether or not it is oriented in the desired direction, by comparing together the direction which has been detected by the magnetic sensor and a direction which has been set in advance, or the like.

As the sensor, a giro which detects the orientation of the container may be employed.

In this case, it is possible always to detect the same fixed direction within the living body. By doing this, it is possible for the inference device to infer whether or not it is oriented in the desired direction; by comparing together the direction which has been detected by the giro and a direction which has been set in advance, or the like.

As the sensor, a gravity sensor which detects the direction of gravity may be employed.

In this case, it is possible always to detect the direction of gravity within the living body by the gravity sensor, irrespective of its own attitude. By this, it is possible for the inference device to infer whether or not it is oriented in the desired direction, by comparing together the direction of gravity and a direction which has been set in advance, or the like.

As the sensor, a luminance sensor which detects the luminance within the living body may be employed.

In this case, since detection of change of luminance within the living body can be detected by the luminance sensor, it is possible for the inference device to infer that shifting toward a desired position within the living body, by comparing together the magnitude of the luminance which has been detected by the luminance sensor and a threshold value which has been set in advance, or by change in the pattern of the luminance, or the like.

As the sensor, a pH sensor which detects the pH value within the living body may be employed.

In this case, since detection of change of the pH value within the living body can be detected by the pH sensor, it is possible for the inference device to infer that shifting toward a desired position within the living body, by comparing together the magnitude of the pH value which has been detected by the pH sensor and a threshold value which has been set in advance, or by change in the pattern of the pH value, or the like.

The second capsule type medical apparatus communication system (which is termed the second capsule type medical apparatus communication system in order to distinguish it from the first capsule type medical apparatus communication system) includes the second capsule type medical apparatus, and an antenna external to the living body, which is disposed outside the living body, and which, along with transmitting a radio wave towards within the living body, also receives the information which is transmitted from the signal transmission device; and, here, the sensor is a signal reception antenna which receives the radio wave which is transmitted from the antenna external to the living body.

In the above described second capsule type medical apparatus communication system, the signal reception antenna receives the radio wave which has been transmitted from the antenna external to the living body, and then sends it to the inference device. The inference device infers the state of communication based upon the magnitude of the radio wave which has been transmitted and has arrived. In other words, since the radio wave is sent out from the antenna external to the living body at a high output level if the capsule type medical apparatus has arrived at a position in which the communication state is satisfactory, or if the matching with the antenna external to the living body is satisfactory, the inference device compares the magnitude of the radio wave which has been sent and has arrived with a threshold value which is set in advance, and infers that the communication state is satisfactory, if it is greater than the threshold value. And, based upon the results of the inference by the inference device, the signal transmission control device operates the signal transmission device to transmit the information to the antenna external to the living body.

By doing this, it is possible to obtain the information from the capsule type medical apparatus in a reliable manner even while it is shifting about within the living body. In particular, it is possible to obtain the information which has been acquired, not all at once at the final end of its acquisition, but during the shifting about within the living body, so that it is possible to obtain the information in a reliable manner outside the living body at any suitable time. By this, even if some problem arises, such as for example that some of the information goes missing or the like, it is possible to reduce the influence of the problem to the minimum limit, since the information up to the point has been obtained in a reliable manner, and accordingly it is possible to enhance the reliability of inspection. Moreover, since it is not necessary for the person such as the patient or the like who is the subject of inspection always to keep the antenna external to his body deployed, and it will be acceptable only to utilize the antenna external to the living body at certain required times, accordingly it is possible to reduce the burden imposed by the inspection upon the person who is the subject of inspection.

The third capsule type medical apparatus communication system includes: the second capsule type medical apparatus; an antenna external to the living body, which is disposed outside the living body, and which receives the information which is transmitted from the signal transmission device; and an energy wave transmission device which is disposes in the vicinity of the antenna external to the living body, and which transmits an energy wave towards within the living body; and, here, the sensor is an energy wave reception device which receives the energy wave which has been transmitted from the energy wave transmission device; and the signal transmission device performs signal transmission using electrical power which has been converted from the energy wave.

With this third capsule type medical apparatus communication system, the energy wave reception device receives the energy wave which has been transmitted from the energy wave transmission device, and then sends it to the inference device. The inference device infers the state of communication based upon the magnitude of the energy wave which has been sent and has arrived. In other words, the inference device compares the magnitude of the energy wave which has been sent and has arrived with a threshold value which is set in advance, and infers that the state of communication is satisfactory if it is greater than the threshold value. And, based upon the results of the inference by the inference device, the signal transmission control device operates the signal transmission device and transmits the information to the antenna external to the living body. By doing this, it is possible to obtain the information in a reliable manner from the capsule type medical apparatus which is shifting about within the living body. Furthermore, since the signal transmission device utilizes the electrical power which has been converted from the energy wave which has been sent and has arrived when transmitting the information, even in the case in which a battery within the capsule type medical apparatus cuts out, it is possible to obtain the information which has been acquired up until that time point in a reliable manner.

The fourth capsule type medical apparatus communication system includes: the second capsule type medical apparatus, in which the sensor is a magnetic sensor; an antenna external to the living body, which is disposed outside the living body, and which receives the information which is transmitted from the signal transmission device; and a magnet which is arranged in a direction which is correlated with a signal reception direction of the antenna external to the living body.

In this fourth capsule type medical apparatus communication system, when the magnetic sensor passes to within the vicinity of where the magnet is disposed, it detects the direction of the magnet, irrespective of its own attitude. By this, the inference device is able, by comparing together the direction of the magnet from the magnetic sensor and a direction which is set in advance, to infer that the signal transmission direction of the signal transmission device is oriented in the direction of the magnet. In other words, since the magnet is correlated with the signal reception direction of the antenna external to the living body, the inference device infers that the signal transmission direction of the signal transmission device is oriented in the signal reception direction of the antenna external to the living body. Accordingly, it is possible to perform signal transmission at a time when the state of communication is satisfactory, and it is possible to obtain the information in a reliable manner from the capsule type medical apparatus which is shifting about within the living body.

The fifth capsule type medical apparatus communication system invention includes: the second capsule type medical apparatus, in which the sensor is a giro; and an antenna external to the living body, which is disposed outside the living body, and which, along with receiving the information which is transmitted from the signal transmission device, also has a signal reception direction in a predetermined direction.

In this fourth capsule type medical apparatus communication system, when it moves within the living body, it is possible always to detect a fixed direction by the giro, irrespective of its own attitude. By comparing together the direction which has been detected by the giro and a direction which is set in advance, the inference device infers that the signal transmission direction of the signal transmission device is oriented in the signal reception direction of the antenna external to the living body, in other words, that the matching with the antenna external to the living body is good, and that the state of communication is in a satisfactory state. Accordingly, it is possible to perform the signal transmission when the state of communication is satisfactory, and it is possible to obtain the information in a reliable manner from the capsule type medical apparatus which is shifting about within the living body.

The sixth capsule type medical apparatus communication system includes: the second capsule type medical apparatus, in which the sensor is a luminance sensor; and an antenna external to the living body, which is disposed outside the living body, and which receives the information which is transmitted from the signal transmission device.

In this sixth capsule type medical apparatus communication system, when it moves about within the living body, the inference device infers that it is positioned at a specific region within the living body from the detected value from the luminance sensor. For example, by taking a luminance value or a pH value which is characteristic of the stomach as a threshold value for which the state of communication is comparatively satisfactory, it may infer that the capsule type medical apparatus is currently positioned within the stomach of the patient. By this, it is possible to obtain the information in a reliable manner from the capsule type medical apparatus which is shifting about within the living body.

In the above described second through sixth capsule type medical apparatus communication systems, the antenna external to the living body may be arranged in a state in which it is separated from the surface of the body by a predetermined interval distance.

In this case, since the antenna external to the living body is kept at the predetermined interval distance from the body of the person undergoing the examination, it is difficult for it to experience any influence due to the impedance of the living body. Accordingly, it is possible to maintain the state of communication with the capsule type medical apparatus within the living body at a more satisfactory state.

Furthermore, the antenna external to the living body may shift in a state in which the interval is maintained.

In this case, since the antenna external to the living body can be shifted in correspondence to the shifting of the capsule type medical apparatus within the living body, and since a single antenna external to the living body can be used for searching the range in which the state of communication is satisfactory over a wide range, accordingly, it is possible to reduce the number of antennas external to the living body which need to be provided, and it is possible to utilize them in a more efficient manner.

Thus, according to the above described second capsule type medical apparatus and the above described second through sixth capsule type medical apparatus communication systems, since, when acquiring the information in the living body, the inference device infers whether or not the state of communication is satisfactory, based upon the magnitude of the radio wave which has been transmitted from the antenna external to the living body, and the signal transmission control device makes the final decision and operates the signal transmission section based upon the result of this inference, therefore, the information is transmitted to the outside of the living body only when the state of communication is satisfactory. Accordingly, it is possible to enhance the reliability of the inspection, since it is possible to obtain the information from within the living body in a reliable manner.

## Claims

1. A capsule type medical apparatus communication system (1), comprising a capsule type medical apparatus (2) adapted to transmit information relating to the body of an examinee from within the body of the examinee to outside it, and an information receiver (3) which is disposed externally to the body of the examinee and which is adapted to receive the information, wherein:
the capsule type medical apparatus (2) is adapted to transmit towards the information receiver (3) a communication confirmation signal which confirms the state of communication with the information receiver (3); and
the information receiver (3) is adapted to, when it has received the communication confirmation signal, transmit a communication authorization signal which authorizes the capsule type medical apparatus (2) to perform communication,
**characterized in that**
the capsule type medical apparatus (2) comprises a communication control means (13) adapted to decide whether or not to transmit the information, based upon the state of reception of the communication authorization signal when the capsule type medical apparatus (2) has received the communication authorization signal; and
the capsule type medical apparatus (2) is adapted to transmit the information towards the information receiver (3) if the communication control means (13) decides to transmit the information.

2. The capsule type medical apparatus communication system (1) according to Claim 1, wherein the information receiver (3) is adapted to perform supply of electrical power to the capsule type medical apparatus (2) with the communication authorization signal.

3. The capsule type medical apparatus communication system (1) according to Claim 1, wherein the information receiver (3) comprises:
a signal reception means (35) adapted to receive, from a capsule type medical apparatus (2) within the body of an examinee, information about the body of the examinee which has been transmitted, and a communication confirmation signal which confirms the state of communication with the capsule type medical apparatus;
a recording section (36) adapted to record the information which has been received;
a communication authorization signal creation section (37) adapted to, when the signal reception means (35) has received the communication confirmation signal, create a communication authorization signal which authorizes the capsule type medical apparatus (2) to transmit the information; and
a communication authorization signal transmission means (38) adapted to transmit the communication authorization signal.

4. The capsule type medical apparatus communication system (1) according to Claim 3, wherein the information receiver (3) is adapted to perform supply of electrical power to the capsule type medical apparatus (2) with the communication authorization signal.

5. The capsule type medical apparatus communication system (1) according to Claim 4, wherein
when the communication confirmation signal is not received, the communication authorization signal transmission means (38) is adapted to transmit the communication authorization signal over an interval which is longer than the signal transmission interval of the communication confirmation signal.

## Patentansprüche

1. Kommunikationssystem (1) für ein Medizingerät vom Kapseltyp mit einem Medizingerät vom Kapseltyp (2), das den Körper eines Patienten betreffende Information vom Inneren des Patientenkörpers nach außen zu übermitteln vermag, und einem Informationsempfänger (3), der außerhalb des Patientenkörpers angeordnet ist und der die Information zu empfangen vermag, wobei:
das Medizingerät vom Kapseltyp (2) dazu ausgebildet ist, ein Kommunikationsrückmeldesignal zum Informationsempfänger (3) zu übermitteln, welches den Status der Kommunikation mit dem Informationsempfänger (3) rückmeldet; und
der Informationsempfänger (3) nach dem Empfangen des Kommunikationsrückmeldesignals ein Kommunikationsberechtigungssignal zu übermitteln vermag, welches das Medizingerät vom Kapseltyp (2) dazu autorisiert, Kommunikation durchzuführen,
**dadurch gekennzeichnet, dass**
das Medizingerät vom Kapseltyp (2) ein Kommunikationssteuerungsmittel (13) aufweist, das, wenn das Medizingerät vom Kapseltyp (2) das Kommunikationsberechtigungssignal empfangen hat, aufgrund des Empfangsstatus des Kommunikationsberechtigungssignals zu entscheiden vermag, ob die Information übermittelt wurde oder nicht; und
das Medizingerät vom Kapseltyp (2) die Information zum Informationsempfänger (3) zu übermitteln vermag, wenn das Kommunikationssteuerungsmittel (13) entscheidet, die Information zu übermitteln.

2. Kommunikationssystem (1) für ein Medizingerät vom Kapseltyp nach Anspruch 1, wobei der Informationsempfänger (3) dazu ausgebildet ist, mit dem Kommunikationsberechtigungssignal die Versorgung des Medizingeräts vom Kapseltyp (2) mit elektrischem Strom durchzuführen.

3. Kommunikationssystem (1) für ein Medizingerät vom Kapseltyp nach Anspruch 1, wobei der Informationsempfänger (3) aufweist:
ein Signalempfangsmittel (35), das dazu ausgebildet ist, von einem Medizingerät vom Kapseltyp (2) innerhalb des Körpers eines Patienten, Information über den Patientenkörper, die übermittelt worden ist, und ein Kommunikationsrückmeldesignal, welches den Status der Kommunikation mit dem Medizingerät vom Kapseltyp rückmeldet, zu empfangen;
einen Aufzeichnungsbereich (36), der die empfangene Information aufzuzeichnen vermag;
einen Erzeugungsbereich für ein Kommunikationsberechtigungssignal (37), der, wenn das Signalempfangsmittel (35) das Kommunikationsrückmeldesignal empfangen hat, ein Kommunikationsberechtigungssignal zu erzeugen vermag, welches das Medizingerät vom Kapseltyp (2) autorisiert, die Information zu übermitteln; und
ein Übertragungsmittel für ein Kommunikationsberechtigungssignal (38), welches das Kommunikationsberechtigungssignal zu übermitteln vermag.

4. Kommunikationssystem (1) für ein Medizingerät vom Kapseltyp nach Anspruch 3, wobei der Informationsempfänger (3) dazu ausgebildet ist, mit dem Kommunikationsberechtigungssignal die Versorgung des Medizingeräts vom Kapseltyp (2) mit elektrischem Strom durchzuführen.

5. Kommunikationssystem (1) für ein Medizingerät vom Kapseltyp nach Anspruch 4, wobei,
wenn das Kommunikationsrückmeldesignal nicht empfangen wird, das Übertragungsmittel für ein Kommunikationsberechtigungssignal (38) das Kommunikationsberechtigungssignal über einen Zeitraum zu übermitteln vermag, der länger ist als das Signalübermittlungszeitintervall des Kommunikationsrückmeldesignals.

## Revendications

1. Système de communication (1) d'appareil médical de type capsule, comprenant un appareil médical de type capsule (2) adapté à transmettre des informations se rapportant au corps d'un sujet examiné de l'intérieur du corps du sujet examiné vers l'extérieur de celui-ci, et un récepteur d'informations (3) qui est disposé à l'extérieur du corps du sujet examiné et qui est adapté à recevoir les informations, dans lequel :
l'appareil médical de type capsule (2) est adapté à transmettre vers le récepteur d'informations (3) un signal de confirmation de communication qui confirme l'état de communication auprès du récepteur d'informations (3) ; et
le récepteur d'informations (3) est adapté à, lorsqu'il a reçu le signal de confirmation de communication, transmettre un signal d'autorisation de communication qui autorise l'appareil médical de type capsule (2) à effectuer une communication,
**caractérisé en ce que**
l'appareil médical de type capsule (2) comprend un moyen de contrôle de communication (13) adapté à décider de transmettre ou non les informations, sur la base de l'état de réception du signal d'autorisation de communication lorsque l'appareil médical de type capsule (2) a reçu le signal d'autorisation de communication ; et
l'appareil médical de type capsule (2) est adapté à transmettre les informations vers le récepteur d'informations (3) si le moyen de contrôle de communication (13) décide de transmettre les informations.

2. Système de communication (1) d'appareil médical de type capsule selon la revendication 1, dans lequel le récepteur d'informations (3) est adapté à effectuer une alimentation en énergie électrique de l'appareil médical de type capsule (2) avec le signal d'autorisation de communication.

3. Système de communication (1) d'appareil médical de type capsule selon la revendication 1, dans lequel le récepteur d'informations (3) comprend :
un moyen de réception de signal (35) adapté à recevoir, d'un appareil médical de type capsule (2) à l'intérieur du corps d'un sujet examiné, des informations se rapportant au corps du sujet examiné qui ont été transmises, et un signal de confirmation de communication qui confirme l'état de communication auprès de l'appareil médical de type capsule ;
une section d'enregistrement (36) adaptée à enregistrer les informations qui ont été reçues ;
une section de création (37) de signal d'autorisation de communication adaptée à, lorsque le moyen de réception de signal (35) a reçu le signal de confirmation de communication, créer un signal d'autorisation de communication qui autorise l'appareil médical de type capsule (2) à transmettre les informations ; et
un moyen de transmission (38) de signal d'autorisation de communication adapté à transmettre le signal d'autorisation de communication.

4. Système de communication (1) d'appareil médical de type capsule selon la revendication 3, dans lequel le récepteur d'informations (3) est adapté à effectuer une alimentation en énergie électrique de l'appareil médical de type capsule (2) avec le signal d'autorisation de communication.

5. Système de communication (1) d'appareil médical de type capsule selon la revendication 4, dans lequel
lorsque le signal de confirmation de communication n'est pas reçu, le moyen de transmission (38) de signal d'autorisation de communication est adapté à transmettre le signal d'autorisation de communication sur un intervalle qui est plus long que l'intervalle de transmission de signal du signal de confirmation de communication.
